# EUROPÄISCHE PATENTSCHRIFT

(11) **EP 1 753 757 B1**
(45) Veröffentlichungstag und Bekanntmachung des Hinweises auf die Patenterteilung: **10.12.2008**
(21) Anmeldenummer: 05747410.8
(22) Anmeldetag: 17.05.2005
(51) Int. Cl.: C07D 413/14, C07D 403/06, C07D 401/14, C07D 401/06, A61K 31/5377, A61K 31/496, A61K 31/4453, A61P 25/04

(54) **SUBSTITUIERTE 2,5-DIAMINOMETHYL-1H-PYRROLE**
SUBSTITUTED 2,5-DIAMINOMETHYL-1H-PYRROLES
2,5-DIAMINOMETHYL-1H-PYRROLES SUBSTITUES

(30) Priorität: 17.05.2004 DE 102004024773
(43) Veröffentlichungstag der Anmeldung: 21.02.2007
(73) Patentinhaber: Grünenthal GmbH, 52078 Aachen (DE)
(72) Erfinder: MERLA, Beatrix, 52078 Aachen (DE); SUNDERMANN, Corinna, 52074 Aachen (DE); JAGUSCH, Utz-Peter, 52066 Aachen (DE); ENGLBERGER, Werner, 52223 Stolberg (DE); HENNIES, Hagen-Heinrich, 52152 Simmerath (DE)
(74) Vertreter: Brosch, Oliver
(86) Internationale Anmeldenummer: PCT/EP2005/005402
(87) Internationale Veröffentlichungsnummer: WO 2005/113549

(56) Entgegenhaltungen:
- EP-A- 0 312 345
- SCOTT M K ET AL: "PIPERAZINYLALKYL HETEROCYCLES AS POTENTIAL ANTIPSYCHOTIC AGENTS" JOURNAL OF MEDICINAL CHEMISTRY, AMERICAN CHEMICAL SOCIETY. WASHINGTON, US, Bd. 38, Nr. 21, 1995, Seiten 4198-4210, XP002092071 ISSN: 0022-2623
- SCOTT M K ET AL: "PYRROLE MANNICH BASES AS POTENTIAL ANTIPSYCHOTIC AGENTS" JOURNAL OF MEDICINAL CHEMISTRY, AMERICAN CHEMICAL SOCIETY. WASHINGTON, US, Bd. 35, Nr. 3, 1992, Seiten 552-558, XP002039583 ISSN: 0022-2623
- HEANEY H ET AL: "The Generation of Iminium Ions Using Chlorosilanes and their Reactions with Electron Rich Aromatic Heterocycles" TETRAHEDRON, ELSEVIER SCIENCE PUBLISHERS, AMSTERDAM, NL, Bd. 53, Nr. 8, 24. Februar 1997 (1997-02-24), Seiten 2941-2958, XP004105380 ISSN: 0040-4020
- MILLAN M J: "Serotonin (5-HT) and pain: A reappraisal of its role in the light of receptor multiplicity" SEMINARS IN THE NEUROSCIENCES, PHILADELPHIA, PA, US, Bd. 7, Nr. 6, Dezember 1995 (1995-12), Seiten 409-419, XP004552144 ISSN: 1044-5765
- CORELLI, F. ET AL: "Studies on antibacterial and antifungal agents. IV. Synthesis and antifungal activity of econazole analogs with a pyrrolic structure Studies on antibacterial and antifungal agents. IV. Synthesis and antifungal activity of econazole analogs with a pyrrolic structure" FARMACO, EDIZIONE SCIENTIFICA , 40(5), 315-24 CODEN: FRPSAX; ISSN: 0430-0920, 1985, XP009052685

## Beschreibung

Die vorliegende Erfindung betrifft substituierte 2,5-Diaminomethyl-1H-pyrrole, Verfahren zu deren Herstellung, Arzneimittel enthaltend diese Verbindungen sowie die Verwendung dieser Verbindungen zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide, wie beispielsweise das Morphin, sind bei der Therapie starker bis sehr starker Schmerzen wirksam, weisen jedoch unerwünschte Begleiterscheinungen auf, wie beispielsweise Atemdepression, Erbrechen, Sedierung oder Obstipation. Nach weiteren schmerzhemmenden Mitteln wird weltweit geforscht.

Scott et al. (J. Med. Chem. 1995, 38, 4198-4210; J. Med. Chem. 1992, 35, 552-558) und EP-A-0 312 345 offenbaren jeweils substituierte Pyrrol-Verbindungen mit antipsychotischer Wirkung.

Heaney et al (Tetrahedron, 1997, 53(8), 2941-2958) offenbart 1,1'-(2-Methylcyclopenta-3,5-dien-1,3-diyl)bis(methylen)dipyrrolidin, 1,1'-(2-Methylcyclopenta-3,5-dien-1,3-diyl)bis(methylen)dipiperidin, 4,4'-(2-Methylcyclopenta-3,5-dien-1,3-diyl)bis(methylen)dimorpholin und *N,N*-Dimethyl(5-methyl-4-(piperidin-1-ylmethyl)cyclopenta-1,3-dienyl)methanamin.

Aufgabe der vorliegenden Erfindung war es daher, neue Wirkstoffe zur Verfügung zu stellen, die sich insbesondere als pharmakologische Wirkstoffe zur Anwendung in Arzneimitteln, vorzugsweise in Arzneimitteln zur Behandlung von Schmerzen eignen.

Diese Aufgabe wurde durch die Bereitstellung der erfindungsgemäßen substituierten 2,5-Diaminomethyl-1H-pyrrole der nachstehenden allgemeinen Formel I gelöst.

Es wurde überraschenderweise gefunden, daß die substituierten 2,5-Diaminomethyl-1 H-pyrrole der nachstehenden allgemeinen Formel I eine hohe Affinität für Opioid-Rezeptoren, insbesondere für µ-Opioid-Rezeptoren, aufweisen und sich daher zur Regulation dieser Rezeptoren eignen.

Ferner eignen sich die erfindungsgemäßen substituierten 2,5-Diaminomethyl-1H-pyrrole der nachstehenden allgemeinen Formel I zur Regulation, vorzugsweise zur Inhibierung des Noradrenalin(NA)-Uptakes sowie zur Regulation, vorzugsweise zur Inhibierung des 5-Hydroxytryptamin-(5-HT)-Uptakes.

Die erfindungsgemäßen substituierten 2,5-Diaminomethyl-1H-pyrrol-Verbindungen der nachstehenden allgemeinen Formel I können daher insbesondere als pharmakologische Wirkstoffe in Arzneimitteln zur Prophylaxe und/oder Behandlung von mit den vorstehend genannten Rezeptoren bzw. Prozessen in Verbindung stehenden Störungen und Erkrankungen eingesetzt werden.

Ein Gegenstand der vorliegenden Erfindung sind daher substituierte 2,5-Diaminomethyl-1H-pyrrole der allgemeinen Formel I, 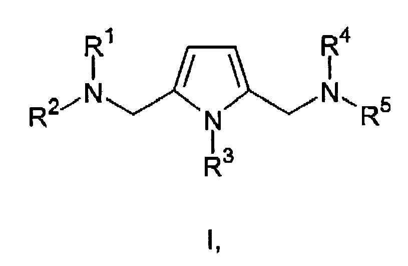 worin
R¹ und R² zusammen mit dem sie verbindenden Stickstoffatom als Ringglied einen gesättigten oder ungesättigten, ggf. wenigstens ein weiteres Heteroatom als Ringglied aufweisenden cycloaliphatischen Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann; wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy substituiert sein kann;
R³ für einen linearen oder verzweigten, ungesättigten oder gesättigten, unsubstituierten oder wenigstens einfach substituierten aliphatischen Rest steht,
R⁴ für einen Wasserstoff-Rest, für einen linearen oder verzweigten, unsubstituierten oder wenigstens einfach substituierten, ungesättigten oder gesättigten aliphatischen Rest, für einen ungesättigten oder gesättigten, unsubstituierten oder wenigstens einfach substituierten, ggf. wenigstens ein Heteroatom als Ringglied aufweisenden cycloaliphatischen Rest, der über eine lineare oder verzweigte Alkylen-Gruppe gebunden sein kann, oder für einen unsubstituierten oder wenigstens einfach substituierten, über eine lineare oder verzweigte Alkylen-Gruppe gebundenen Aryl-Rest steht,
R⁵ für einen linearen oder verzweigten, unsubstituierten oder wenigstens einfach substituierten, ungesättigten oder gesättigten aliphatischen Rest, für einen ungesättigten oder gesättigten, unsubstituierten oder wenigstens einfach substituierten, ggf. wenigstens ein Heteroatom als Ringglied aufweisenden cycloaliphatischen Rest, der über eine lineare oder verzweigte Alkylen-Gruppe gebunden sein kann, oder für einen unsubstituierten oder wenigstens einfach substituierten, über eine lineare oder verzweigte Alkylen-Gruppe gebundenen Aryl-Rest steht,
oder
R⁴ und R⁵ zusammen mit dem sie verbindenden Stickstoffatom als Ringglied einen gesättigten oder ungesättigten, ggf. wenigstens ein weiteres Heteroatom als Ringglied aufweisenden cycloaliphatischen Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl,-NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann; wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy substituiert sein kann;
wobei die vorstehend genannten aliphatischen Reste mit Substituenten unabhängig voneinander ausgewählt aus der Gruppe bestehend aus Halogen, Hydroxy, -CN, - CF₃, -CHF₂, -CH₂F, C₁₋₆-Alkoxy und ggf. wenigstens einfach substituiertem Phenyl, dessen Substituenten jeweils unabhängig voneinander ausgewählt werden können aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂. -CH₂F, C₁₋₅-Alkyl und C₁₋₅-Alkoxy, substituiert sein können;
wobei die vorstehend genannten Aryl-Reste mit Substituenten unabhängig voneinander ausgewählt werden aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein können, wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂ -NO₂, -CN, -CF₃, -CHF₂, - CH₂F, C₁₋₅-Alkyl und C₁₋₅-Alkoxy substituiert sein kann;
wobei die vorstehend genannten cycloaliphatischen Reste in Position der Reste R⁴ und R⁵ mit Substituenten unabhängig voneinander ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein können, wobei der jeweilige Furanyl-, Thiophenyl-, Pyridinyl-, Phenyl- oder Benzyl-Substituent selbst unsubstituiert oder mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, - NO₂, -CN, -CF₃, -CHF₂, -CH₂F, C₁₋₅-Alkyl und C₁₋₅-Alkoxy substituiert sein kann;
jeweils gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate,
wobei die Verbindungen der allgemeinen Formel I, in denen
die Reste R¹ und R² sowie die Reste R⁴ und R⁵ jeweils zusammen mit dem sie verbindenden Stickstoffatom als Ringglied den gleichen Rest ausgewählt aus der Gruppe bestehend aus Pyrrolidinyl-, Morpholinyl- und Piperidinyl- bilden, und der Rest R³ jeweils für eine Methyl-Gruppe steht, d.h. die Verbindungen 2,5-bis(N-piperidinyl-methyl)-1-methylpyrrol, 2,5-bis(N-morpholinyl-methyl)-1-methylpyrrol und 2,5-bis(N-pyrrolidinyl-methyl)-1-methylpyrrol,
sowie Verbindungen der allgemeinen Formel I, in denen
die Reste R¹ und R² zusammen mit dem sie verbindenden Stickstoffatom als Ringglied einen Piperidinyl-Rest bilden und die Reste R³ bis R⁵ jeweils für eine Methyl-Gruppe stehen,
ausgenommen sind.

Ebenfalls bevorzugt können Verbindungen der allgemeinen Formel I, in denen
die Reste R¹ und R² sowie die Reste R⁴ und R⁵ jeweils zusammen mit dem sie verbindenden Stickstoffatom als Ringglied den gleichen Rest ausgewählt aus der Gruppe bestehend aus Pyrrolidinyl-, Morpholinyl- und Piperidinyl- bilden, und der Rest R³ jeweils für eine Methyl-Gruppe steht, d.h. die Verbindungen 2,5-bis(N-piperidinyl-methyl)-1-methylpyrrol, 2,5-bis(N-morpholinyl-methyl)-1-methylpyrrol und 2,5-bis(N-pyrrolidinyl-methyl)-1-methylpyrrol,
sowie Verbindungen der allgemeinen Formel I, in denen
die Reste R¹ und R² zusammen mit dem sie verbindenden Stickstoffatom als Ringglied einen Piperidinyl-Rest bilden und die Reste R³ bis R⁵ jeweils für eine Methyl-Gruppe stehen,
und ggf. jeweils deren entsprechende Salze ausgenommen sein.

Bevorzugt sind erfindungsgemäße substituierte 2,5-Diaminomethyl-1H-pyrrole der allgemeinen Formel I, in denen die Reste R¹ und R² zusammen mit dem sie verbindenden Stickstoffatom als Ringglied einen gesättigten oder ungesättigten, ggf. wenigstens ein weiteres Heteroatom als Ringglied aufweisenden 4-, 5-, 6-, 7-, 8-oder 9-gliedrigen cycloaliphatischen Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann,
bevorzugt zusammen mit dem sie verbindenden Stickstoffatom als Ringglied einen gesättigten oder ungesättigten, ggf. wenigstens ein weiteres Heteroatom ausgewählt aus der Gruppe bestehend aus Stickstoff, Sauerstoff und Schwefel als Ringglied aufweisenden 4-, 5-, 6-, 7-, 8- oder 9-gliedrigen cycloaliphatischen Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann,
besonders bevorzugt zusammen mit dem sie verbindenden Stickstoffatom als Ringglied einen Imidazolidinyl-, Aziridinyl-, Azetidinyl-, Pyrrolidinyl-, Piperidinyl-, Azepanyl-, Azokanyl-, Piperazinyl-, Morpholinyl- oder Thiomorpholinyl-Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, O-C₁₋₅-Alkyl, Cl, F, Br, I, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann;
wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, - NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy substituiert sein kann;
und die Reste R³ bis R⁵ jeweils die vorstehend genannte Bedeutung haben, jeweils gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Weiterhin bevorzugt sind erfindungsgemäße substituierte 2,5-Diaminomethyl-1H-pyrrole der allgemeinen Formel I, in denen der Rest R³ für einen linearen oder verzweigten, gesättigten oder ungesättigten, unsubstituierten oder wenigstens einfach substituierten aliphatischen C₁₋₁₀-Rest steht, wobei die Substituenten unabhängig voneinander ausgewählt werden können aus der Gruppe bestehend aus Halogen, Hydroxy, -CN, -CF₃, -CHF₂, -CH₂F, C₁₋₆-Alkoxy und ggf. wenigstens einfach substituiertem Phenyl, dessen Substituenten jeweils unabhängig voneinander ausgewählt werden können aus der Gruppe bestehend aus Hydroxy, F, Cl, Br, I, - NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy,
bevorzugt für einen linearen oder verzweigten, unsubstituierten oder wenigstens einfach substituierten C₁₋₅-Alkyl-Rest steht, wobei die Substituenten unabhängig voneinander ausgewählt werden können aus der Gruppe bestehend aus Halogen, Hydroxy, -CN, -CF₃, -CHF₂, -CH₂F, C₁₋₆-Alkoxy und ggf. wenigstens einfach substituiertem Phenyl, dessen Substituenten jeweils unabhängig voneinander ausgewählt werden können aus der Gruppe bestehend aus Hydroxy, F, Cl, Br, I, - NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy,
besonders bevorzugt für einen Alkyl-Rest ausgewählt aus der Gruppe bestehend aus Methyl, Ethyl, n-Propyl, iso-Propyl, *n*-Butyl, iso-Butyl, sec-Butyl und *tert*-Butyl steht und die Reste R¹, R², R⁴ und R⁵ jeweils die vorstehend genannte Bedeutung haben, jeweils gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Des weiteren sind erfindungsgemäße substituierte 2,5-Diaminomethyl-1H-pyrrole der allgemeinen Formel I, in denen der Rest R⁴ für einen Wasserstoff-Rest, für einen linearen oder verzweigten, gesättigten oder ungesättigten, unsubstituierten oder wenigstens einfach substituierten aliphatischen C₁₋₁₀-Rest, wobei die Substituenten unabhängig voneinander ausgewählt werden können aus der Gruppe bestehend aus Halogen, Hydroxy, -CN, -CF₃, -CHF₂, -CH₂F, C₁₋₆-Alkoxy und ggf. wenigstens einfach substituiertem Phenyl, dessen Substituenten jeweils unabhängig voneinander ausgewählt werden können aus der Gruppe bestehend aus Hydroxy, F, Cl, Br, I, - NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₆-Alkoxy, für einen gesättigten oder ungesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte C₁₋₃-Alkylen-Gruppe gebunden sein kann, oder für einen unsubstituierten oder wenigstens einfach substituierten, über eine lineare oder verzweigte C₁₋₃-Alkylen-Gruppe gebundenen 5- bis 14-gliedrigen Aryl-Rest steht,
bevorzugt für einen Wasserstoff-Rest, für einen linearen oder verzweigten C₁₋₅-Alkyl-Rest, für einen gesättigten oder ungesättigten 4-, 5-, 6-, 7-, 8- oder 9-gliedrigen cycloaliphatischen Rest, der über eine lineare oder verzweigte C₁₋₃-Alkylen-Gruppe gebunden sein kann, oder für einen unsubstituierten oder wenigstens einfach substituierten, über eine lineare oder verzweigte C₁₋₃-Alkylen-Gruppe gebundenen Phenyl-Rest steht,
besonders bevorzugt für einen Wasserstoff-Rest, für einen Alkyl-Rest ausgewählt aus der Gruppe bestehend aus Methyl, Ethyl, n-Propyl, iso-Propyl, *n*-Butyl, iso-Butyl, sec-Butyl und *tert*-Butyl oder für einen Benzyl-Rest steht; wobei die vorstehend genannten Aryl-Reste mit Substituenten unabhängig voneinander ausgewählt werden aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein können, wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂ -NO₂, -CN, -CF₃, -CHF₂, - CH₂F, C₁₋₅-Alkyl und C₁₋₅-Alkoxy substituiert sein kann;
wobei die vorstehend genannten cycloaliphatischen Reste mit Substituenten unabhängig voneinander ausgewählt aus der Gruppe bestehend aus C₁₋₅-Alkyl, - C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein können, wobei der jeweilige Furanyl-, Thiophenyl-, Pyridinyl-, Phenyl- oder Benzyl-Substituent selbst unsubstituiert oder mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, - CH₂F, C₁₋₅-Alkyl und C₁₋₅-Alkoxy substituiert sein kann und die Reste R¹ bis R³, R⁵ sowie R⁴ und R⁵ zusammen jeweils die vorstehend genannte Bedeutung haben, jeweils gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Ferner sind erfindungsgemäße substituierte 2,5-Diaminomethyl-1H-pyrrole der allgemeinen Formel I bevorzugt, in denen der Rest R⁵ für einen linearen oder verzweigten, gesättigten oder ungesättigten, unsubstituierten oder wenigstens einfach substituierten aliphatischen C₁₋₁₀-Rest, wobei die Substituenten unabhängig voneinander ausgewählt werden können aus der Gruppe bestehend aus Halogen, Hydroxy, -CN, -CF₃, -CHF₂, -CH₂F, C₁₋₅-Alkoxy und ggf. wenigstens einfach substituiertem Phenyl, dessen Substituenten jeweils unabhängig voneinander ausgewählt werden können aus der Gruppe bestehend aus Hydroxy, F, Cl, Br, I, - NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy, für einen gesättigten oder ungesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte C₁₋₃-Alkylen-Gruppe gebunden sein kann, oder für einen unsubstituierten oder wenigstens einfach substituierten, über eine lineare oder verzweigte C₁₋₃-Alkylen-Gruppe gebundenen 5- bis 14-gliedrigen Aryl-Rest steht, bevorzugt für einen linearen oder verzweigten C₁₋₅-Alkyl-Rest, für einen gesättigten oder ungesättigten 4-, 5-, 6-, 7-, 8- oder 9-gliedrigen cycloaliphatischen Rest, der über eine lineare oder verzweigte C₁₋₃-Alkylen-Gruppe gebunden sein kann, oder für einen unsubstituierten oder wenigstens einfach substituierten, über eine lineare oder verzweigte C₁₋₃-Alkylen-Gruppe gebundenen Phenyl-Rest steht,
besonders bevorzugt für einen Alkyl-Rest ausgewählt aus der Gruppe bestehend aus Methyl, Ethyl, n-Propyl, iso-Propyl, n-Butyl, iso-Butyl, sec-Butyl und *tert*-Butyl oder für einen Benzyl-Rest steht;
wobei die vorstehend genannten Aryl-Reste mit Substituenten unabhängig voneinander ausgewählt werden aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein können, wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂ -NO₂, -CN, -CF₃, -CHF₂, - CH₂F, C₁₋₅-Alkyl und C₁₋₅-Alkoxy substituiert sein kann;
wobei die vorstehend genannten cycloaliphatischen Reste mit Substituenten unabhängig voneinander ausgewählt aus der Gruppe bestehend aus C₁₋₅-Alkyl, - C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf, wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein können, wobei der jeweilige Furanyl-, Thiophenyl-, Pyridinyl-, Phenyl- oder Benzyl-Substituent selbst unsubstituiert oder mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, - CH₂F, C₁₋₅-Alkyl und C₁₋₅-Alkoxy substituiert sein kann;
und die Reste R¹ bis R⁴ sowie R⁴ und R⁵ zusammen jeweils die vorstehend genannte Bedeutung haben, jeweils gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Weiterhin sind erfindungsgemäße substituierte 2,5-Diaminomethyl-1H-pyrrole der allgemeinen Formel I bevorzugt, in denen die Reste R⁴ und R⁵ zusammen mit dem sie verbindenden Stickstoffatom als Ringglied einen gesättigten oder ungesättigten, ggf. ein Heteroatom als Ringglied aufweisenden 4-, 5-, 6-, 7-, 8- oder 9-gliedrigen cycloaliphatischen Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann,
bevorzugt zusammen mit dem sie verbindenden Stickstoffatom als Ringglied einen gesättigten oder ungesättigten, ggf. wenigstens ein weiteres Heteroatom ausgewählt aus der Gruppe bestehend aus Stickstoff, Sauerstoff und Schwefel als Ringglied aufweisenden 4-, 5-, 6-, 7-, 8- oder 9-gliedrigen cycloaliphatischen Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -C(=O)-O-C₁₋₅-Alkyl, - O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann,
besonders bevorzugt zusammen mit dem sie verbindenden Stickstoffatom als Ringglied einen Imidazolidinyl-, Aziridinyl-, Azetidinyl-, Pyrrolidinyl-, Piperidinyl-, Azepanyl-, Azokanyl-, Piperazinyl-, Morpholinyl- oder Thiomorpholinyl-Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann; wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy substituiert sein kann;
und die Reste R¹ bis R³ sowie R⁴ und R⁵ getrennt voneinander jeweils die vorstehend genannte Bedeutung haben, jeweils gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Sofern einer oder mehrere der Reste R³ bis R⁵ für einen linearen oder verzweigten, gesättigten oder ungesättigten aliphatischen Rest, d. h. für einen linearen oder verzweigten Alkyl-, Alkenyl- oder Alkinyl-Rest stehen, der einfach oder mehrfach, beispielsweise 1-, 2-, 3-, 4- oder 5-fach, substituiert ist, können diese Substituenten unabhängig voneinander ausgewählt werden aus der Gruppe bestehend aus Halogen, Hydroxy, -CN, -CF₃, -CHF₂, -CH₂F, C₁₋₆-Alkoxy und ggf. wenigstens einfach substituiertem Phenyl, besonders bevorzugt aus der Gruppe bestehend aus F, Cl, Br und Hydroxy. Erfindungsgemäß können die aliphatischen Reste nur solche Substituenten aufweisen, die über eine Einfachbindung gebunden sind, d.h. mehrwertige Substituenten wie beispielsweise eine Oxo-Gruppe (=O) sind nicht umfaßt.

Sofern der vorstehend genannte Phenylsubstituent selbst einfach oder mehrfach, beispielsweise 1-, 2-, 3-, 4- oder 5-fach, substituiert ist, können dessen Substituenten jeweils unabhängig voneinander ausgewählt werden aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, - CH₂F, C₁₋₅-Alkyl und C₁₋₅-Alkoxy.

Als geeignete Alkyl-, Alkenyl- und Alkinyl-Reste, die einfach oder mehrfach, beispielsweise 1-, 2-, 3-, 4- oder 5-fach, substituiert sein können, seien beispielsweise Methyl, Ethyl, n-Propyl, iso-Propyl, n-Butyl, iso-Butyl, sec-Butyl, tert-Butyl, n-Pentyl, iso-Pentyl, neo-Pentyl, n-Hexyl, 2-Hexyl, n-Heptyl, n-Octyl, n-Nonyl, n-Decyl, -C(H)(C₂H₅)₂, -C(H)(n-C₃H₇)₂, -CH₂-CH₂-C(H)(CH₃)-(CH₂)₃-CH₃, Vinyl, Ethinyl, Propenyl, Allyl, Propinyl, Butenyl, Butinyl, Pentenyl, Pentinyl, Hexenyl und Hexinyl genannt.

Der von R¹ und R² zusammen mit dem sie verbindenden Stickstoffatom als Ringglied gebildete, monozyklische, ggf. wenigstens ein weiteres Heteroatom als Ringglied aufweisende cycloaliphatische Rest ist gesättigt oder ungesättigt, nicht aber aromatisch. Er kann einfach oder mehrfach, beispielsweise 1-, 2-, 3-, 4- oder 5-fach, gleich oder verschieden substituiert sein, wobei die jeweiligen Substituenten unabhänig voneinander ausgewählt werden können aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, - NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl.
Der jeweilige Furanyl-, Thiophenyl-, Pyridinyl-, Phenyl- oder Benzyl-Substituent selbst kann unsubstituiert oder einfach oder mehrfach, ggf. 1-, 2-, 3-, 4- oder 5-fach substituiert sein, wobei dessen Substituenten unabhängig voneinander ausgewählt werden können aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy.

Der von R⁴ und R⁵ zusammen mit dem sie verbindenden Stickstoffatom als Ringglied gebildete, monozyklische, ggf. wenigstens ein weiteres Heteroatom als Ringglied aufweisende cycloaliphatische Rest ist gesättigt oder ungesättigt, nicht aber aromatisch. Er kann einfach oder mehrfach, beispielsweise 1-, 2-, 3-, 4- oder 5-fach, gleich oder verschieden substituiert sein, wobei die Substituenten unabhängig voneinander ausgewählt werden können aus der Gruppe bestehend aus C₁₋₅-Alkyl, - C(=O)-O-C₁₋₅-Alkyl, -O-C₁₋₆-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl.
Der jeweilige Furanyl-, Thiophenyl-, Pyridinyl-, Phenyl- oder Benzyl-Substituent selbst kann unsubstituiert oder einfach oder mehrfach, ggf. 1-, 2-, 3-, 4- oder 5-fach, substituiert sein, wobei dessen Substituenten unabhängig voneinander ausgewählt werden können aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy.

Sofern die Reste R¹ und R² und/oder R⁴ und R⁵ zusammen mit dem sie verbindenden Stickstoffatom als Ringglied einen cycloaliphatischen Rest mit wenigstens einem weiteren Heteroatom bilden, können die Heteroatome, sofern nicht anders angegeben, bevorzugt ausgewählt werden aus der Gruppe bestehend aus Sauerstoff, Stickstoff und Schwefel. Vorzugsweise können die zyklischen Reste 1 oder 2 weitere Heteroatome als Ringglieder aufweisen.

Sofern die Reste R¹ und R² und/oder R⁴ und R⁵ zusammen mit dem sie verbindenden Stickstoffatom als Ringglied einen ggf. wenigstens ein weiteres Heteroatom als Ringglied aufweisenden cycloaliphatischen Rest bilden, der einfach oder mehrfach substituiert sein kann, kann dieser bevorzugt ausgewählt werden aus der Gruppe bestehend aus Imidazolinyl, Aziridinyl, Azetidinyl, Pyrrolidinyl, Piperidinyl, Azepanyl, Azokanyl, Piperazinyl, Morpholinyl und Thiomorpholinyl.

Sofern einer oder beide der vorstehend genannten Reste R⁴ und R⁵ für einen gesättigten oder ungesättigten, ggf. wenigstens ein weiteres Heteroatom, beispielsweise 1 oder 2 Heteroatome, als Ringglied aufweisenden cycloaliphatischen Rest steht (stehen) oder einen solchen Rest aufweist (aufweisen), der einfach oder mehrfach, beispielsweise 1-, 2-, 3-, 4- oder 5-fach, gleich oder verschieden substituiert ist, so können die entsprechenden Substituenten unabhängig voneinander ausgewählt werden aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl.
Der jeweilige Furanyl-, Thiophenyl-, Pyridinyl-, Phenyl- oder Benzyl-Substituent selbst kann unsubstituiert oder einfach oder mehrfach, ggf. 1-, 2-, 3-, 4- oder 5-fach, substituiert sein, wobei dessen Substituenten unabhängig voneinander ausgewählt werden können aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy. Die Heteroatome können, sofern nicht anders angegeben, bevorzugt ausgewählt werden aus der Gruppe bestehend aus Sauerstoff, Stickstoff und Schwefel.

Als geeignete cycloaliphatische Reste, die einfach oder mehrfach, beispielsweise 1-, 2-, 3-, 4- oder 5-fach, substituiert sein können, seinen beispielsweise Cyclopropyl, Cylobutyl, Cyclopentyl, Cyclohexyl, Cyclohexyl, Cyclooctyl, Cyclononyl, Cyclopentenyl, Cyclohexenyl, Cycloheptenyl oder Cyclooctenyl genannt.

Als geeignete, eines oder mehrere Heteroatome als Ringglied aufweisende cycloaliphatische Reste, die einfach oder mehrfach, ggf. 1-, 2-, 3-, 4- oder 5-fach, substituiert sein können, seinen beispielsweise Imidazolinyl, Aziridinyl, Azetidinyl, Pyrrolidinyl, Piperidinyl, Azepanyl, Azokanyl, Piperazinyl, Tetrahydrofuranyl (Tetrahydrofuryl), Morpholinyl und Thiomorpholinyl genannt.

Sofern einer oder beide der vorstehend genannten Reste R⁴ und R⁵ für einen einfach oder mehrfach, beispielsweise 1-, 2-, 3-, 4- oder 5-fach, substituierten Aryl-Rest steht (stehen) oder einen solchen Rest aufweist (aufweisen), so können die entsprechenden Substituenten unabhängig voneinander ausgewählt werden aus der Gruppe bestehend aus C₁₋₅-Alkyl, -C(=O)-O-C₁₋₅-Alkyl, -O-C₁₋₅-Alkyl, Cl, F, Br, I, - C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl. Der jeweilige Furanyl-, Thiophenyl-, Pyridinyl-, Phenyl- oder Benzyl-Substituent selbst kann unsubstituiert oder einfach oder mehrfach, ggf. 1-, 2-, 3-, 4- oder 5-fach, substituiert sein, wobei dessen Substituenten unabhängig voneinander ausgewählt werden können aus der Gruppe bestehend aus Hydroxy, F, Cl, Br, I, -NH₂, -NH-C₁₋₅-Alkyl, -N(C₁₋₅-Alkyl)₂, - NO₂, -CN, -CF₃, -CHF₂, -CH₂F, C₁₋₅-Alkyl und C₁₋₅-Alkoxy.

Als geeignete Aryl-Reste, die einfach oder mehrfach substituiert sein können, seien beispielsweise Phenyl, 1-Naphthyl und 2-Naphthyl genannt.

Die vorstehend genannten C₁₋₅-Alkoxy- und C₁₋₅-Alkyl-Reste können jeweils linear oder verzweigt sein. Die C₁₋₅-Alkyl-Reste umfassen die Reste Methyl, Ethyl, n-Propyl, iso-Propyl, n-Butyl, iso-Butyl, sec-Butyl, tert-Butyl, n-Pentyl, iso-Pentyl und neo-Pentyl, die C₁₋₅-Alkoxy-Reste umfassen die Reste Methoxy, Ethoxy, n-Propoxy, iso-Propoxy, n-Butoxy, iso-Butoxy, sec-Butoxy, tert-Butoxy, n-Pentoxy, iso-Pentoxy und neo-Pentoxy.

Ganz besonders bevorzugt sind erfindungsgemäße substituierte 2,5-Diaminomethyl-1 H-pyrrole ausgewählt aus der Gruppe bestehend aus
Diethyl-(1-methyl-5-piperidin-1-ylmethyl-1H-pyrrol-2-ylmethyl)-amin,
1-(1-Methyl-5-piperidin-1-ylmethyl-1H-pyrrol-2-ylmethyl)-4-methylpiperidin,
4-[1-Methyl-5-(4-phenyl-piperidin-1-ylmethyl)-1H-pyrrol-2-ylmethyl]-morpholin,
1-(1-Methyl-5-piperidin-1-ylmethyl-1H-pyrrol-2-ylmethyl)-4-phenyl-piperazin,
1-[5-(4-Benzyl-piperidin-1-ylmethyl)-1-methyl-1H-pyrrol-2-ylmethyl]-azepan,
Benzyl-[5-(4-benzyl-piperidin-1-ylmethyl)-1-methyl-1H-pyrrol-2-ylmethyl]-methylamin,
4-[5-(4-Benzyl-piperidin-1-ylmethyl)-1-methyl-1H-pyrrol-2-ylmethyl]-morpholin,
(5-Azepan-1-ylmethyl-1-methyl-1H-pyrrol-2-ylmethyl)-benzyl-methyl-amin,
Benzyl-methyl-[1-methyl-5-(4-phenyl-piperidin-1-ylmethyl)-1H-pyrrol-2-ylmethyl]-amin und
Benzyl-methyl-[1-methyl-5-(4-phenyl-piperidin-1-ylmethyl)-1H-pyrrol-2-ylmethyl]-amin,
jeweils ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.
Ein weiterer Gegenstand der vorliegenden Erfindung ist ein Verfahren zur Herstellung der erfindungsgemäßen substituierten 2,5-Diaminomethyl-1H-pyrrole der allgemeinen Formel I, gemäß dem ein substituiertes Aminomethyl-1H-pyrrol der allgemeinen Formel II, 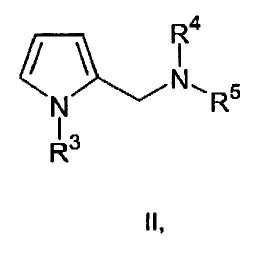 worin die Reste R³, R⁴ und R⁵ jeweils die vorstehend genannte Bedeutung haben, nach üblichen, dem Fachmann bekannten Methoden, vorzugsweise in einem geeigneten Reaktionsmedium, wie z.B. CH₂Cl₂, CH₃CN, Dimethylformamid (DMF) oder Mischungen aus wenigstens zwei dieser Komponenten, bei Raumtemperatur (ca. 20-25°C) mit einem Iminiumsalz der allgemeinen Formel III, 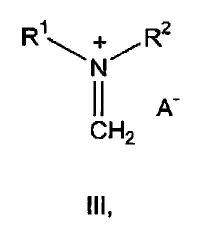 worin R¹ bis R² die vorstehend genannte Bedeutung haben und A⁻ für ein geeignetes Anion, vorzugsweise für Cl⁻, AlCl₄⁻, Br⁻, I⁻ oder CF₃-SO₃⁻ (Triflat-Anion) steht, zu einem erfindungsgemäßen substituierten 2,5-Dimethylamino-1 H-pyrrol der allgemeinen Formel I umgesetzt und dieses ggf. nach üblichen, dem Fachmann bekannten Methoden, vorzugsweise durch Extraktion, gereinigt und ggf. isoliert wird.

Die Verbindungen der allgemeinen Formel II können nach üblichen, dem Fachmann bekannten Methoden, beispielsweise aus den am Markt käuflich erhältlichen Reagenzien der allgemeinen Formel IV, 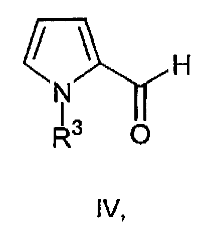 hergestellt werden, wie z.B. in A. F. Abdel-Magid et al., Journal of Organic Chemistry 1996, 61, Seiten 3849-3862 beschrieben. Die entsprechende Literaturbeschreibung wird hiermit als Referenz eingeführt und gilt als Teil der Offenbarung.

Die Iminiumsalze der allgemeinen Formel III können ebenfalls nach üblichen, dem Fachmann bekannten Verfahren, beispielsweise aus den entsprechenden Aminalen der nachstehenden allgemeinen Formel V, 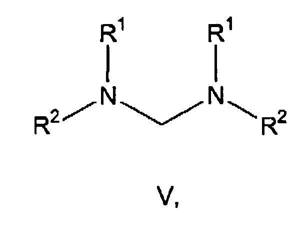 worin die Reste R¹ und R² die vorstehend genannte Bedeutung haben, erhalten werden, wie beispielsweise in H. Heaney, Tetrahedron 1997, 53, Seiten 2941-2958 und H. Heaney, Tetrahedron Lett. 1988, 29, Seiten 2377-2380 beschrieben. Die entsprechenden Literaturbeschreibungen werden hiermit als Referenz eingeführt und gelten als Teil der Offenbarung.

Auch die Herstellung der Aminale der allgemeinen Formel V kann nach literaturbekannten Methoden erfolgen, wie z.B. in H. Heaney, Tetrahedron 1997, 53, Seiten 2941-2958 und H. Heaney, Tetrahedron Lett. 1988, 29, Seiten 2377-2380 beschrieben. Die entsprechenden Literaturbeschreibungen werden hiermit als Referenz eingeführt und gelten als Teil der Offenbarung.
Die erfindungsgemäßen substituierten 2,5-Diaminomethyl-1H-pyrrole der allgemeinen Formel I sowie entsprechende Stereoisomere können sowohl in Form ihrer freien Basen, ihrer freien Säuren wie auch in Form entsprechender Salze, insbesondere physiologisch verträglicher Salze, isoliert werden.

Die freien Basen der jeweiligen erfindungsgemäßen substituierten 2,5-Diaminomethyl-1H-pyrrole der allgemeinen Formel I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in die entsprechenden Salze, vorzugsweise physiologisch verträglichen Salze, überführt werden.

Die freien Basen der jeweiligen erfindungsgemäßen substituierten 2,5-Diaminomethyl-1H-pyrrole der allgemeinen Formel 1 sowie entsprechender Stereoisomere können bevorzugt durch Versetzen der in einem geeigneten organischen Lösungsmittel, wie z.B. Butan-2-on (Methylethylketon), gelösten Verbindungen der allgemeinen Formel I oder entsprechender Stereoisomere als freie Basen mit Trimethylsilylchlorid (TMSCI) in die entsprechenden Hydrochlorid-Salze übergeführt werden.

Die freien Basen der jeweiligen substituierten 2,5-Diaminomethyl-1H-pyrrole der allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der substituierten 2,5-Diaminomethyl-1H-pyrrole der allgemeinen Formel I und entsprechender Stereoisomere durch Umsetzung mit einer geeigneten Base in die entsprechenden physiologisch verträglichen Salze überführt werden.

Die erfindungsgemäßen substituierten 2,5-Diaminomethyl-1H-pyrrole der allgemeinen Formel I und entsprechende Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise ihrer Hydrate, erhalten werden.

Sofern die erfindungsgemäßen substituierten 2,5-Diaminomethyl-1H-pyrrole der allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten 2,5-Diaminomethyl-1H-pyrrole der allgemeinen Formel I einschließlich der vorstehend ausgenommenen Verbindungen und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung sind daher Arzneimittel enthaltend wenigstens ein erfindungsgemäßes substituiertes 2,5-Diaminomethyl-1H-pyrrol der allgemeinen Formel I einschließlich der vorstehend ausgenommenen Verbindungen, gegebenenfalls in Form seines Racemates, seiner reinen Stereoisomeren, insbesondere Enantiomeren oder Diastereomeren, oder in Form von Mischungen der Stereoisomeren, insbesondere der Enantiomeren und/oder Diastereomeren, in einem beliebigen Mischungsverhältnis, oder jeweils in Form eines entsprechenden Salzes, insbesondere des physiologisch verträglichen Salzes, besonders bevorzugt des Hydrochlorid-Salzes, oder eines entsprechenden Solvates, insbesondere des Hydrates, sowie ggf. einen oder mehrere physiologisch verträgliche Hilfsstoffe.

Diese erfindungsgemäßen Arzneimittel eignen sich insbesondere zur Opioid-Rezeptor-Regulation, vorzugsweise zur µ-Opioid-Rezeptor-Regulation, zur Regulation des Noradrenalin(NA)-Uptakes, vorzugsweise zur Inhibierung des Noradrenalin(NA)-Uptakes, sowie zur Regulation des 5-Hydroxytryptamin-(5-HT)-Uptakes, vorzugsweise zur Inhibierung des 5-Hydroxytryptamin-(5-HT)-Uptakes.

Ebenfalls bevorzugt eignen sich die erfindungsgemäßen Arzneimittel zur Prophylaxe und/oder Behandlung von Störungen oder Krankheiten, die zumindest teilweise durch Opioid-Rezeptoren, insbesondere durch µ-Opioid-Rezeptoren, und/oder Noradrenalin(NA)-Rezeptoren und/oder 5-Hydroxytryptamin-(5-HT)-Rezeptoren vermittelt werden.

Ebenfalls bevorzugt eignen sich die erfindungsgemäßen Arzneimittel zur Behandlung von Schmerzen, vorzugsweise ausgewählt aus der Gruppe bestehend aus chronischen Schmerzen und/oder akuten Schmerzen und/oder neuropathischen Schmerzen, zur Prophylaxe und/oder Behandlung von Entzugserscheinungen, Gedächtnisstörungen, neurodegenerativen Erkrankungen, vorzugsweise ausgewählt aus der Gruppe bestehend aus Morbus Parkinson, Morbus Huntington, Morbus Alzheimer und Multipler Sklerose, Epilepsie, cardiovaskulären Störungen, Wasserspeicher-Krankheiten, intestinaler Motilität (Diarrhoe), Harninkontinenz, Anorexie, Tinnitus, Pruritus, Depressionen, sexueller Dysfunktionen, vorzugsweiser erektiler Dysfunktion oder Atemwegserkrankungen, Störungen der Nahrungsmittelaufnahme, vorzugsweise ausgewählt aus der Gruppe bestehend aus Fettsucht, Bulimie, Anorexie, Kachexie und Typ II Diabetes (nicht-insulinabhängiger Diabetes), oder zur Anxiolyse, zur Diurese, zur Unterdrückung des Miktionsreflexes, zur Reduzierung des Suchtpotentials von Opioiden, vorzugsweise Morphin, zur Modulation der Bewegungsaktivität, zur Beeinflussung des cardiovaskulären Systems, vorzugsweise zur Vasodilatation der Arterien, oder zur Regulation des Elektrolyt-Haushaltes.

Besonders bevorzugt eignen sich die erfindungsgemäßen Arzneimittel zur Behandlung von Schmerzen, vorzugsweise ausgewählt aus der Gruppe bestehend aus chronischen Schmerzen, akuten Schmerzen und neuropathischen Schmerzen. Ein weiterer Gegenstand der vorliegenden Erfindung ist die Verwendung eines oder mehrerer substituierter 2,5-Diaminomethyl-1H-pyrrole der allgemeinen Formel I einschließlich der vorstehend ausgenommenen Verbindungen, gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze, insbesondere der physiologisch verträglichen Salze, besonders bevorzugt der Hydrochloride, oder jeweils in Form entsprechender Solvate, insbesondere der Hydrate, zur Herstellung eines Arzneimittels zur Opiod-Rezeptor-Regulation, vorzugsweise zur µ-Opioid-Rezeptor-Regulation, zur Regulation des Noradrenalin(NA)-Uptakes, vorzugsweise zur Inhibierung des Noradrenalin(NA)-Uptakes oder zur Regulation des Hydroxytryptamin-(5-HT)-Uptakes, vorzugsweise zur Inhibierung des 5-Hydroxytryptamin-(5-HT)-Uptakes.

Ein weiterer Gegenstand der vorliegenden Erfindung ist die Verwendung eines oder mehrerer substituierter 2,5-Diaminomethyl-1H-pyrrole der allgemeinen Formel I einschließlich der vorstehend ausgenommenen Verbindungen, gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze, insbesondere der physiologisch verträglichen Salze, besonders bevorzugt der Hydrochloride, oder jeweils in Form entsprechender Solvate, insbesondere der Hydrate, zur Herstellung eines Arzneimittels zur Behandlung von Schmerzen, vorzugsweise ausgewählt aus der Gruppe bestehend aus chronischen Schmerzen, Schmerzen und neuropathischen Schmerzen, zur Prophylaxe und/oder Behandlung von Entzugserscheinungen, Gedächtnisstörungen, neurodegenerativen Erkrankungen, vorzugsweise ausgewählt aus der Gruppe bestehend aus Morbus Parkinson, Morbus Huntington, Morbus Alzheimer und/oder multipler Sklerose, Epilepsie, cardiovaskulärer Störungen, Wasserspeicher-Krankheiten, intestinaler Motilität (Diarrhoe), Harninkontinenz, Anorexie, Tinnitus, Pruritus, Depressionen, sexueller Dysfunktionen, vorzugsweiser erektiler Dysfunktion oder Atemwegserkrankungen, Störungen der Nahrungsmittelaufnahme, vorzugsweise ausgewählt aus der Gruppe bestehend aus Fettsucht, Bulimie, Anorexie, Kachexie und Typ II Diabetes (nicht-insulinabhängiger Diabetes), oder zur Anxiolyse, zur Diurese, zur Unterdrückung des Miktionsreflexes, zur Reduzierung des Suchtpotentials von Opioiden, vorzugsweise Morphin, zur Modulation der Bewegungsaktivität, zur Beeinflussung des cardiovaskulären Systems, vorzugsweise zur Vasodilatation der Arterien, oder zur Regulation des Elektrolyt-Haushaltes

Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben einem oder mehreren substituierten 2,5-Diaminomethyl-1H-pyrrolen der allgemeinen Formel I einschließlich der vorstehend ausgenommenen Verbindungen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form eines entsprechenden Salzes, insbesondere eines physiologisch verträglichen Salzes, oder in Form eines entsprechenden Solvates, insbesondere des Hydrates, enthalten die erfindungsgemäßen Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll.

Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Substituierte 2,5-Diaminomethyl-1H-pyrrole der allgemeinen Formel I einschließlich der vorstehend ausgenommenen Verbindungen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form eines entsprechenden Salzes, insbesondere eines physiologisch verträglichen Salzes, oder in Form eines entsprechenden Solvates, insbesondere des Hydrates, in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweiligen substituierten 2,5-Diaminomethyl-1H-pyrrole der allgemeinen Formel I einschließlich der vorstehend ausgenommenen Verbindungen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form eines entsprechenden Salzes, insbesondere eines physiologisch verträglichen Salzes, oder in Form eines entsprechenden Solvates, insbesondere des Hydrates, auch verzögert freisetzen.

Die Herstellung der erfindungsgemäßen Arzneimittel erfolgt mit Hilfe von üblichen, dem Fachmann bekannten Mitteln, Vorrichtungen, Methoden und Verfahren, wie sie beispielsweise in A.R. Gennaro (Hrsg.), Remington's Pharmaceutical Sciences, 17. Edition, Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge des jeweiligen substituierten 2,5-Dimethylamino-1H-pyrrols der allgemeinen Formel I einschließlich der vorstehend ausgenommenen Verbindungen, gegebenenfalls in Form des Racemates, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form eines entsprechenden Salzes, insbesondere eines physiologisch verträglichen Salzes, oder jeweils in Form eines entsprechenden Solvates, insbesondere des Hydrates, kann variieren und ist beispielsweise abhängig vom Gewicht oder Alter des Patienten sowie von der Applikationsart, der Indikation und dem Schweregrad der Erkrankung. Üblicherweise werden 0,005 bis 500 mg/kg, vorzugsweise 0,05 bis 50 mg/kg Körpergewicht des Patienten wenigstens eines substituierten 2,5-Dimethylamino-1H-pyrrols der allgemeinen Formel I einschließlich der vorstehend ausgenommenen Verbindungen, gegebenenfalls in Form seines Racemates, seines reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form eines entsprechenden Salzes, insbesondere eines physiologisch verträglichen Salzes, oder in Form eines entsprechenden Solvates, insbesondere des Hydrates, appliziert.

### Pharmakologische Methoden:

### a) Methode zur Bestimmung der Affinität zum humanen µ-Opioidrezeptor

Die Rezeptoraffinität zum humanen µ-Opioidrezeptor wird in einem homogenen Ansatz in Mikrotiterplatten bestimmt. Hierzu werden Verdünnungsreihen des jeweils zu prüfenden substituierten 2,5-Dimethylamino-1H-pyrrols der allgemeinen Formel I mit einer Rezeptormembranpräparation (15-40 µg Protein pro 250 µl Inkubationsansatz) von CHO-K1-Zellen, welche den humanen µ-Opioidrezeptor (µ-Opioat-Rezeptor) exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wird 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wird zusätzlich 25 µmol/l Naloxon zugegeben. Nach Beendigung der neunzigminütigen Inkubationszeit werden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wird die prozentuale Verdrängung des radioaktiven Liganden aus seiner Bindung zum humanen µ-Opiatrezeptor bei einer Konzentration der zu prüfenden Verbindungen von 1 µmol/l bestimmt und als Prozent Hemmung der spezifischen Bindung angegeben. Ausgehend von der prozentualen Verdrängung durch unterschiedliche Konzentrationen der zu prüfenden Verbindungen der allgemeinen Formel I wurden IC₅₀ Hemmkonzentrationen berechnet, die eine 50-prozentige Verdrängung des radioaktiven Liganden bewirken. Durch Umrechnung mittels der Cheng-Prusoff-Beziehung lassen sich Kᵢ-Werte für die Prüfsubstanzen erhalten.

### b) Methode zur Bestimmung der Noradrenalin- und der 5HT-Uptake-Inhibierung:

Für in vitro Studien werden Synaptosomen aus Rattenhirnarealen frisch isoliert, wie in der Veröffentlichung "The isolation of nerve endings from brain" von E.G. Gray und V.P. Whittaker, J. Anatomy 96, Seiten 79-88, 1962, beschrieben. Die entsprechende Literaturbeschreibung wird hiermit als Referenz eingeführt und gilt als Teil der Offenbarung.

Das Gewebe (Hypothalamus für die Bestimmung der Noradrenalin-Uptake-Inhibierung und Mark und Pons für die Bestimmung der 5HT-Uptake-Inhibierung) wird in eisgekühlter 0,32 M Sucrose (100 mg Gewebe / 1ml) in einem Glas-Homogenisierer mit Teflonstößel homogenisiert, indem fünf volle Auf- und Abschläge bei 840 Umdrehungen /Minute benutzt werden.

Das Homogenat wird bei 4°C für 10 Minuten bei 1000 g zentrifugiert. Nach anschließender Zentrifugierung bei 17000 g für 55 Minuten erhält man die Synaptosomen (P₂-Fraktion), die in 0,32 M Glucose (0,5 mL/ 100 mg des ursprünglichen Gewichts) noch einmal suspendiert werden.
Der jeweilige Uptake wird in einer 96-well Mikrotiterplatte gemessen. Das Volumen beträgt 250 µl und die Inkubation erfolgt bei Raumtemperatur (ca. 20-25 °C) unter O₂ Atmosphäre.

Die Inkubationszeit beträgt 7,5 Minuten für [³H]-NA und 5 Minuten für [³H]-5-HT. Anschließend werden die 96 Proben durch eine Unifilter GF/B^{®} Mikrotiterplatte (Packard) filtriert und mit 200 ml inkubierten Puffer mit Hilfe eines "Brabdel Cell-Harvester MPXRI-96T" gewaschen. Die Unifilter GF/B Platte wird bei 55°C 1 Stunde getrocknet. Im Anschluß wird die Platte mit einem Back seal^{®} (Packard) verschlossen und mit 35 µl Szintilationsflüssigkeit pro Well (Ultima Gold^{®}, Packard) versetzt. Nach dem Verschließen mit einem top seal^{®} (Packard) wird, nach der Einstellung des Gleichgewichts (etwa 5 Stunden), die Radioaktivität in einem "Trilux 1450 Microbeta" (Wallac, Freiburg, Deutschland) bestimmt.

Die Menge des bei der vorstehenden Bestimmung eingesetzten Proteins entspricht den aus der Literatur bekannten Werten, wie z.B. in "Protein measurement with the folin phenol reagent", Lowry et al., J. Biol. Chem., 193, 265-275,1951 beschrieben.

Eine detaillierte Methodenbeschreibung kann ferner auch der Literatur, beispielsweise aus M.Ch. Frink, H.-H. Hennies, W. Engelberger, M. Haurand und B. Wilffert (1996) Arzneim.-Forsch./Drug Res. 46 (III), 11, 1029-1036, entnommen werden.

Die entsprechenden Literaturbeschreibungen werden hiermit jeweils als Referenz eingeführt und gelten als Teil der vorliegenden Offenbarung.

Folgende Kenndaten wurden für den NA- bzw. 5-HT-Transporter ermittelt:

| | |
|---|---|
| NA-Uptake : | Km = 0,32 ± 0,11 µM |
| | |
| 5HT-Uptake : | Km = 0,084 ± 0,011 µM |

### c) Untersuchung auf analgetische Wirksamkeit im Writhing-Test

Die Untersuchung der erfindungsgemäßen Verbindungen der allgemeinen Formel auf analgetische Wirksamkeit wird im Phenylchinon-induzierten Writhing an der Maus, modifiziert nach I.C. Hendershot und J. Forsaith (1959) J. Pharmacol. Exp. Ther. 125, 237-240 durchgeführt. Die entsprechende Literaturbeschreibung wird hiermit als Referenz eingeführt und gilt als Teil der Offenbarung.

Dazu werden männliche NMRI-Mäuse mit einem Gewicht von 25 bis 30 g verwendet. Gruppen von 10 Tieren pro Verbindungsdosis erhalten 10 Minuten nach intravenöser Gabe der zu untersuchenden Verbindungen 0,3 ml/Maus einer 0.02%igen wäßrigen Lösung von Phenylchinon (Phenylbenzochinon, Fa. Sigma, Deisenhofen, Deutschland; Herstellung der Lösung unter Zusatz von 5 Gew.-% Ethanol und Aufbewahrung im Wasserbad bei 45°C) intraperitoneal appliziert. Die Tiere werden einzeln in Beobachtungskäfige gesetzt. Mit Hilfe eines Drucktastenzählers wird die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten. Alle Verbindungen werden in der Standarddosierung von 10 mg/kg getestet.

Im folgenden wird die Erfindung anhand von Beispielen erläutert. Diese Erläuterungen sind lediglich beispielhaft und schränken den allgemeinen Erfindungsgedanken nicht ein.

### Beispiele:

Die Messung der NMR-Spektren erfolgte auf einem Gerät vom Typ Bruker DPX 300 für die 300 MHz-Spektren und auf einem Gerät vom Typ Bruker DRX 600 für die 600 MHz-Spektren.

Die jeweiligen Chemikalien und Lösungsmittel wurden kommerziell von den üblichen Herstellern erworben.

### A)

### Allgemeine Synthesevorschrift zur Herstellung von Verbindungen der allgemeinen Formel II:

Der jeweilige Pyrrolcarbaldehyd (90 mmol) der allgemeinen Formel IV wurde in 600 ml Tetrahydrofuran gelöst und nacheinander mit dem entsprechenden Amin der allgemeinen Formel HNR⁴R⁵ (90 mmol) und Natriumborhydridtriacetat (NaBH(OAc)₃ (126 mmol)) versetzt. Nach 20 Stunden Rühren bei Raumtemperatur (ca. 20-25 °C) wurde das Reaktionsgemisch am Rotationsverdampfer eingeengt. Der so erhaltene Rückstand wurde in 500 ml Wasser und 200 ml Diethylether aufgenommen und mit Eisessig (30 ml) auf pH 4-5 eingestellt. Es wurde dreimal mit je 100 ml Diethylether extrahiert. Anschließend wurde die wäßrige Phase mit gesättigter Natriumhydrogencarbonat-Lösung auf pH 8 eingestellt und fünfmal mit je 100 ml Diethylether extrahiert. Die organische Phase wurde über Magnesiumsulfat getrocknet und am Rotationsverdampfer eingeengt.

Die nach der vorstehenden allgemeinen Synthesevorschrift hergestellten Verbindungen der allgemeinen Formel II sind in der nachfolgenden Tabelle 1 wiedergegeben:

**Tabelle 1. Verbindungen der allgemeinen Formel II**

| Verbindungen | R⁴ | R⁵ | R³ | Ausbeute [%] |
|---|---|---|---|---|
| **II-1** | ]-(CH₂)₂-O-(CH₂)₂-[ | | CH₃ | 87 |
| **II-2** | ]-(CH₂)₅-[ | | CH₃ | 70 |
| **II-3** | CH₂CH₃ | CH₂CH₃ | CH₃ | 55 |
| **II-4** | CH₂Phenyl | CH₃ | CH₃ | 33 |
| **II-5** | ]-(CH₂)₆-[ | | CH₃ | 61 |

Die Struktur der Verbindungen **II-1** bis **II-5** wurde jeweils mit Hilfe der ¹H-NMR-Spektroskopie bestimmt. Die NMR-Daten der Verbindungen **II-1** bis **II-3** stimmen mit den aus der Literatur bekannten Daten wie in W. Herz et al. Journal of the American Chemical Society 1951, 73, Seiten 4921-4923 angegeben überein. Im folgenden sind die chemischen Verschiebungen ausgewählter Verbindungen wiedergegeben.

### II-4 Benzyl-methyl-(1-methyl-1H-pyrrol-2-ylmethyl)-amin

δ (DMSO, 300 MHz) = 2,11 (s, 3 H, N(C*H*₃)CH₂Ph); 3,41 - 3,44 (m, 2 H, N(CH₃)C*H*₂Ph); 3,45 - 3,48 (m, 2 H, -C*H*₂-N-); 5,99 - 6,03 (m, 2 H, N(CH₃)-]-C*H*C*H*CHC-[); 6,53 - 6,58 (m, 1 H, N(CH₃)-]-CHCHC*H*C-[); 7,17 - 7,32 (m, 5 H, Ph).

### II-5 1-(1-Methyl-1H-pyrrol-2-ylmethyl)-azepan

δ (DMSO, 300 MHz) = 1,52 -1,65 (m, 8 H, N(CH₂)₂(C*H*₂)₂(C*H*₂)₂); 2,51 - 2,60 (m, 4 H, N(C*H*₂)₂(CH₂)₂(CH₂)₂); 3,49 - 3.52 (m, 2 H, -C*H*₂-N-); 3,62 (s, 3 H, N-CH₃); 5,91-5,98 (m, 1 H, N(CH₃)-]-C*H*C*H*CHC-[); 5,99 - 6,04 (m, 1 H, N(CH₃)-]-C*H*C*H*CHC-[); 6,53 - 6,61 (m, 1 H, N(CH₃)-]-CHCHC*H*C-[).

### B)

### Allgemeine Arbeitsvorschrift für die Synthese der erfindungsgemäßen Verbindungen der allgemeinen Formel I

In einem Kolben wurden äquimolare Mengen des jeweiligen Iminiumsalzes der allgemeinen Formel III und des jeweiligen Aminomethyl-1 H-pyrrols der allgemeinen Formel II in Dichlormethan vorgelegt und für 16 Stunden bei Raumtemperatur gerührt. Zur Aufarbeitung wurde zunächst mit HCl-Lösung angesäuert und die nichtbasischen Verunreinigungen mit Diethylether oder tert-Butylmethylether extrahiert. Anschließend wurde die wäßrige Phase mit Na₂CO₃-Lösung neutralisiert und das Produkt mit Diethylether oder tert-Butylmethylether extrahiert. Nach dem Trocknen der organischen Phase über Magnesiumsulfat erhielt man das Produkt, das zur weiteren Aufreinigung mit Hilfe ethanolischer HCl-Lösung in das Dihydrochlorid übergeführt wurde, das mehrmals mit kaltem Ethanol gewaschen wurde.

Das folgende Beispiel der erfindungsgemäßen Verbindungen der allgemeinen Formel I wurden so hergestellt:

### Beispiel 1:

### Diethyl-(1-methyl-5-piperidin-1-ylmethyl-1H-pyrrol-2-ylmethyl)-amin Dihydrochlorid

δ (DMSO, 600 MHz) = 1,23 - 1,31 (m, 6 H, N(CH₂C*H*₃)₂, 1,33 - 1,41 (m, 1 H, N(CH₂CH₂)₂C*H*₂); 1,64 - 1,72 (m, 1 H, N(CH₂CH₂)₂C*H*₂); 1,73 - 1,86 (m, 4 H, N(CH₂C*H*₂)₂CH₂); 2,86 - 2,94 (m, 2 H, N(C*H*₂CH₂)₂CH₂); 3,02 - 3,17 (m, 4 H, N(C*H*₂CH₃)₂, N(C*H*₂CH₃)₂); 3,26 - 3,41 (m, 2 H, N(C*H*₂CH₂)₂CH₂); 3,78 (s, 3 H, NC*H*₃); 4,24 - 4,35 (m, 4 H, C*H*₂N(CH₂C*H*₂)₂CH₂, C*H*₂N(CH₂CH₃)₂); 6,36 - 6,46 (m, 2 H, N(CH₃)]-CC*H*C*H*C-[);10,19 (s, 1 H, HCl); 10,26 (s, 1 H, HCl).

Allgemeine Arbeitsvorschrift für die automatisierte Synthese der erfindungsgemäßen Verbindungen der allgemeinen Formel I. Die Synthese erfolgte auf einer automatisierten Syntheseanlage der Firma Zymark.

| | | |
|---|---|---|
| Ansatzmengen: | • | 200 µmol Pyrrol der allgemeinen Formel II |
| | • | 200 µmol Iminiumsalz der allgemeinen Formel II |
| | | |
| Pipettiervolumina: | • | 1 ml Pyrrollösung |
| | • | 1 ml Iminiumsalzlösung |
| | | |
| Stammlösungen: | • | 0,2 M Pyrrollösung in MeCN (Lösung I) |
| | • | 0,2 M Iminiumsalzlösung in DMF (Lösung II) |

### Synthesedurchführung:

In ein trockenes Gewindeglas mit Septumkappe wurden bei 20 °C 200 µmol Iminiumsalz der allgemeinen Formel III (Lösung II, 1 ml) vorgelegt und mit 200 µmol Pyrrol-Derivat (Lösung I, 1 ml) versetzt. Die Reaktionslösung wurde 16 Stunden bei 18°C gerührt. Anschließend wurden bei 20°C 2 ml HCl-Lösung (0.1 M) und 2 ml Dichlormethan zugegeben. Die Reaktionslösung wurde 30 Minuten im Spin-Reaktor durchmischt. Der Rührfisch wurde entfernt und das Gefäß mit 2 ml Dichlormethan ausgespült.

### Syntheseaufarbeitung:

Die organische Phase wurde abgenommen und verworfen. Im Vortexer wurden 4 ml Dichlormethan zugegeben und anschließend weitere 10 Minuten im Spinreaktor durchmischt. Die organische Phase wurde nach dem Zentrifugieren wieder abgenommen und verworfen, die wäßrige Phase wurde nochmals mit 4 ml Dichlormethan versetzt und mit 0,7 ml 7.5%-iger NaHCO₃-Lösung auf pH 8-9 eingestellt. Die Lösung wurde im Spinreaktor intensiv durchmischt, und nach dem Zentrifugieren wurde die organische Phase abgetrennt und gesammelt. Die wäßrige Phase wurde analog noch einmal mit 4 ml Dichlormethan extrahiert. Anschließend wurden die vereinigten, organischen Phasen über eine MgSO₄-Kartusche getrocknet und unter vermindertem Druck eingeengt.

Die folgenden Beispiele der erfindungsgemäßen Verbindungen der allgemeinen Formel I wurden entsprechend hergestellt:

### Beispiel 2:

*1-(1-Methyl-5-piperidin-lymethyl-1H-pyrrol-2-ylmethyl)-4-methylpiperidin*
MS: (ESI) m/z = 290 [M⁺ + 1]; 205; 191; 108.

### Beispiel 3:

*4-[1-Methyl-5-(4-phenyl-piperidin-1-ylmethyl)-1H-pyrrol-2-ylmethyl]-morpholin*
MS: (ESI) m/z = 355 [M⁺ + 1]; 193; 108.

### Beispiel 4:

*1-(1-Methyl-5-piperidin-1-ylmethyl-1H-pyrrol-2-ylmethyl)-4-phenyl-piperazin*
MS: (ESI) m/z = 353 [M⁺ + 1]; 268; 190; 108.

### Beispiel 5:

*1-[5-(4-Bengyl-piperidin-1-ylmethyl)-1-methyl-1H-pyrrol-2-ylmethyl]-azepan*
MS: (ESI) m/z = 380 [M⁺ + 1]; 281; 205; 108.

### Beispiel 6:

*Benzyl-[5-(4-benzyl-piperidin-1-ylmethyl)-1-methyl-1H-pyrrol-2-ylmethyl]-methyl-amin*
MS: (ESI) m/z = 402 [M⁺ + 1]; 281; 227

### Beispiel 7:

*4-[5-(4-Benzyl-piperidin-1-ylmethyl)-1-methyl-1H-pyrrol-2-ylmethyl]-morpholin*
MS: (ESI) m/z = 368 [M⁺ + 1]; 281; 193 108.

### Beispiel 8:

*(5-Azepan-1-ylmethyl-1-methyl-1H-pyrro*/*-2-ylmethyl)-benzyl-methyl-amin*
MS: (ESI) m/z = 326 [M⁺ + 1]; 227; 205; 108.

### Beispiel 9:

*Benzyl-methyl-[1-methyl-5-(2-methyl-piperidin-1-ylmethyl)-1H-pyrrol-2-ylmethyl]-amin*
MS: (ESI) m/z = 326 [M⁺ + 1]; 227; 204; 108.

### Beispiel 10:

*Benzyl-methyl-[1-methyl-5-(4-phenyl-piperidin-1-ylmethyl)-1H-pyrrol-2-ylmethyl]-amin*
MS: (ESI) m/z = 326 [M⁺ + 1]; 227; 108.

### Pharmakologische Daten:

### b) 5-HT-Uptake-Inhibierung und Noradrenalin(NA)-Wiederaufnahmehemmung

Die 5-HT-Uptake-Inhibierung und Noradrenalin-Uptake-Inhibierung der erfindungsgemäßen 2,5-Dimethylamino-1 H-pyrrole der allgemeinen Formel I wurde wie vorstehend beschrieben bestimmt. Die Werte für einige ausgewählte Verbindungen sind in der nachfolgenden Tabelle 2 wiedergegeben.

**Tabelle 2:**

| Verbindung gemäß Beispiel | R¹ | R² | R⁴ | R⁵ | R³ | 5HT- Uptake Hemmung [2] | NA-Uptake Hemmung [3] |
|---|---|---|---|---|---|---|---|
| **1** | ]-(CH₂)₅-[ | | C₂H₅ | C₂H₅ | CH₃ | 34 | |
| **2** | ]-(CH₂)₅-[-4-CH₃ | | ]-(CH₂)₅-[ | | CH₃ | 72 | |
| **3** | ]-(CH₂)₄-[-4N-Phenyl | | ]-(CH₂)₂O(CH₂)₂-[ | | CH₃ | 59 | 71 |
| **4** | ]-(CH₂)₄-[-4N-Phenyl | | ]-(CH₂)₅-[ | | CH₃ | 48 | 77 |
| **5** | ]-(CH₂)₅-[-4-CH₂Phenyl | | ]-(CH₂)₆-[ | | CH₃ | 64 | 100 |
| **6** | ]-(CH₂)₅-[-4-CH₂Phenyl | | CH₃ | CH₂Phenyl | CH₃ | 61 | 100 |
| **7** | ]-(CH₂)₅-[-4-CH₂Phenyl | | ]-(CH₂)₂O(CH₂)₂-[ | | CH₃ | 55 | 100 |
| **8** | ]-(CH₂)₆-[ | | CH₃ | CH₂Phenyl | CH₃ | | 62 |
| **9** | ]-(CH₂)₅-[-2-CH₃ | | CH₃ | CH₂Phenyl | CH₃ | | 57 |
| **10** | ]-(CH₂)₅-[-4-Phenyl | | CH₃ | CH₂Phenyl | CH₃ | | 53 |

| | | | | | | | |
|---|---|---|---|---|---|---|---|
| [2] 5-HT-Uptake, Ratte, 10 µm, % Hemmung, [3] NA-Uptake, Ratte, 10 µM Hemmung. | | | | | | | |

## Patentansprüche

1. Substituierte 2,5-Diaminomethyl-1H-pyrrole der allgemeinen Formel I, 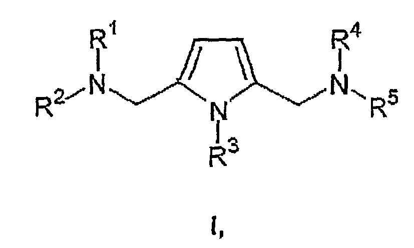 worin
R¹ und R² zusammen mit dem sie verbindenden Stickstoffatom als Ringglied einen gesättigten oder ungesättigten, ggf. wenigstens ein weiteres Heteroatom als Ringglied aufweisenden cycloaliphatischen Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann; wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy substituiert sein kann;
R³ für einen linearen oder verzweigten, ungesättigten oder gesättigten, unsubstituierten oder wenigstens einfach substituierten aliphatischen Rest steht,
R⁴ für einen Wasserstoff-Rest, für einen linearen oder verzweigten, unsubstituierten oder wenigstens einfach substituierten, ungesättigten oder gesättigten aliphatischen Rest, für einen ungesättigten oder gesättigten, unsubstituierten oder wenigstens einfach substituierten, ggf. wenigstens ein Heteroatom als Ringglied aufweisenden cycloaliphatischen Rest, der über eine lineare oder verzweigte Alkylen-Gruppe gebunden sein kann, oder für einen unsubstituierten oder wenigstens einfach substituierten, über eine lineare oder verzweigte Alkylen-Gruppe gebundenen Aryl-Rest steht,
R⁵ für einen linearen oder verzweigten, unsubstituierten oder wenigstens einfach substituierten, ungesättigten oder gesättigten aliphatischen Rest, für einen ungesättigten oder gesättigten, unsubstituierten oder wenigstens einfach substituierten, ggf. wenigstens ein Heteroatom als Ringglied aufweisenden cycloaliphatischen Rest, der über eine lineare oder verzweigte Alkylen-Gruppe gebunden sein kann, oder für einen unsubstituierten oder wenigstens einfach substituierten, über eine lineare oder verzweigte C₁₋₃-Alkylen-Gruppe gebundenen Aryl-Rest steht,
oder
R⁴ und R⁵ zusammen mit dem sie verbindenden Stickstoffatom als Ringglied einen gesättigten oder ungesättigten, ggf. wenigstens ein weiteres Heteroatom als Ringglied aufweisenden cycloaliphatischen Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann; wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Sr, I, -NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy substituiert sein kann;
wobei die vorstehend genannten aliphatischen Reste mit Substituenten unabhängig voneinander ausgewählt aus der Gruppe bestehend aus Halogen, Hydroxy, -CN, -CF₃, -CHF₂, -CH₂F, C₁₋₆-Alkoxy und ggf. wenigstens einfach substituiertem Phenyl, dessen Substituenten jeweils unabhängig voneinander ausgewählt werden können aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy, substituiert sein können;
wobei die vorstehend genannten Aryl-Reste mit Substituenten unabhängig voneinander ausgewählt werden aus der Gruppe bestehend aus C₁₋₅-Alkyl, - C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein können, wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂-NO₂, -CN, -CF₃, -CHF₂, - CH₂F, C₁₋₅-Alkyl und C₁₋₅-Alkoxy substituiert sein kann;
wobei die vorstehend genannten cycloaliphatischen Reste in Position der Reste R⁴ und R⁵ mit Substituenten unabhängig voneinander ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl,-ggf. wenigstens einfach substituiertem Phenyl und ggf. wenigstens einfach substituiertem Benzyl substituiert sein können, wobei der jeweilige Furanyl-, Thiophenyl-, Pyridinyl-, Phenyl- oder Benzyl-Substituent selbst unsubstituiert oder mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, - NO₂, -CN, -CF₃, -CHF₂, -CH₂F, C₁₋₅-Alkyl und C₁₋₅-Alkoxy substituiert sein kann;
jeweils gegebenenfalls in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze oder jeweils in Form entsprechender Solvate,
mit der Maßgabe, daß Verbindungen der allgemeinen Formel I, in denen
die Reste R¹ und R² sowie die Reste R⁴ und R⁵ jeweils zusammen mit dem sie verbindenden Stickstoffatom als Ringglied den gleichen Rest ausgewählt aus der Gruppe bestehend aus Pyrrolidinyl-, Morpholinyl- und Piperidinyl- bilden, und der Rest R³ jeweils für eine Methyl-Gruppe steht, sowie
die Reste R¹ und R² zusammen mit dem sie verbindenden Stickstoffatom als Ringglied einen Piperidinyl-Rest bilden und die Reste R³ bis R⁵ jeweils für eine Methyl-Gruppe stehen,
ausgenommen sind.

2. Verbindungen gemäß Anspruch 1, **dadurch gekennzeichnet, daß** die Reste R¹ und R² zusammen mit dem sie verbindenden Stickstoffatom als Ringglied einen gesättigten oder ungesättigten, ggf. wenigstens ein weiteres Heteroatom als Ringglied aufweisenden 4-, 5-, 6-, 7-, 8- oder 9-gliedrigen cycloaliphatischen Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(CH₁₋₅-Alkyl)₂. ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann, bevorzugt zusammen mit dem sie verbindenden Stickstoffatom als Ringglied einen gesättigten oder ungesättigten, ggf. wenigstens ein weiteres Heteroatom ausgewählt aus der Gruppe bestehend aus Stickstoff, Sauerstoff und Schwefel als Ringglied aufweisenden 4-, 5-, 6-, 7-, 8- oder 9-gliedrigen cycloaliphatischen Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann,
besonders bevorzugt zusammen mit dem sie verbindenden Stickstoffatom als Ringglied einen Imidazolidinyl-, Aziridinyl-, Azetidinyl-, Pyrrolidinyl-, Piperidinyl-, Azepanyl-, Azokanyl-, Piperazinyl-, Morpholinyl- oder Thiomorpholinyl-Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann;
wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, - CH₂F, C₁₋₅-Alkyl und C₁₋₅-Alkoxy substituiert sein kann.

3. Verbindungen gemäß Anspruch 1 oder 2, **dadurch gekennzeichnet, daß** der Rest R³ für einen linearen oder verzweigten, gesättigten oder ungesättigten, unsubstituierten oder wenigstens einfach substituierten aliphatischen C₁₋₁₀-Rest steht, wobei die Substituenten unabhängig voneinander ausgewählt werden können aus der Gruppe bestehend aus Halogen, Hydroxy, -CN, -CF₃, -CHF₂, -CH₂F, C₁₋₆-Alkoxy und ggf. wenigstens einfach substituiertem Phenyl, dessen Substituenten jeweils unabhängig voneinander ausgewählt werden können aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy,
bevorzugt für einen linearen oder verzweigten, unsubstituierten oder wenigstens einfach substituierten C₁₋₅-Alkyl-Rest steht, wobei die Substituenten unabhängig voneinander ausgewählt werden können aus der Gruppe bestehend aus Halogen, Hydroxy, -CN, -CF₃, -CHF₂, -CH₂F, C₁₋₆-Alkoxy und ggf. wenigstens einfach substituiertem Phenyl, dessen Substituenten jeweils unabhängig voneinander ausgewählt werden können aus der Gruppe bestehend aus Hydroxy, F, Cl, Br, I, -NH₂, -NH-C₁₋₅-Alkyl, - N(C₁₋₅-Alkyl)₂, -NO₂, -CN, -CF₃, -CHF₂, -CH₂F, C₁₋₅-Alkyl und C₁₋₅-Alkoxy,
besonders bevorzugt für einen Alkyl-Rest ausgewählt aus der Gruppe bestehend aus Methyl, Ethyl, *n*-Propyl, iso-Propyl, *n*-Butyl, iso-Butyl, sec-Butyl und *tert*-Butyl steht.

4. Verbindungen gemäß einem oder mehreren der Ansprüche 1 bis 3, **dadurch gekennzeichnet, daß** der Rest R⁴ für einen Wasserstoff-Rest, für einen linearen oder verzweigten, gesättigten oder ungesättigten, unsubstituierten oder wenigstens einfach substituierten aliphatischen C₁₋₁₀-Rest, wobei die Substituenten unabhängig voneinander ausgewählt werden können aus der Gruppe bestehend aus Halogen, Hydroxy, -CN, -CF₃, -CHF₂, -CH₂F, C₁₋₆-Alkoxy und ggf. wenigstens einfach substituiertem Phenyl, dessen Substituenten jeweils unabhängig voneinander ausgewählt werden können aus der Gruppe bestehend aus Hydroxy, F, Cl, Br, I, -NH₂, -NH-C₁₋₅-Alkyl, - N(C₁₋₅-Alkyl)₂, -NO₂, -CN, -CF₃, -CHF₂, -CH₂F, C₁₋₅-Alkyl und C₁₋₅-Alkoxy, für einen gesättigten oder ungesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte C₁₋₃₋Alkylen-Gruppe gebunden sein kann, oder für einen unsubstituierten oder wenigstens einfach substituierten, über eine lineare oder verzweigte C₁₋₃-Alkylen-Gruppe gebundenen, 5- bis 14-gliedrigen Aryl-Rest steht,
bevorzugt für einen Wasserstoff-Rest, für einen linearen oder verzweigten C₁₋₅-Alkyl-Rest, für einen gesättigten oder ungesättigten 4-, 5-, 6-, 7-, 8- oder 9-gliedrigen cycloaliphatischen Rest, der über eine lineare oder verzweigte C₁₋₃-Alkylen-Gruppe gebunden sein kann, oder für einen unsubstituierten oder wenigstens einfach substituierten, über eine lineare oder verzweigte C₁₋₃-Alkylen-Gruppe gebundenen Phenyl-Rest steht,
besonders bevorzugt für einen Wasserstoff-Rest, für einen Alkyl-Rest ausgewählt aus der Gruppe bestehend aus Methyl, Ethyl, Propyl, iso-Propyl, *n*-Butyl, iso-Butyl, sec-Butyl und *tert*-Butyl oder für einen Benzyl-Rest steht;
wobei die vorstehend genannten Aryl-Reste mit Substituenten unabhängig voneinander ausgewählt werden aus der Gruppe bestehend aus C₁₋₅-Alkyl, - C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und 99f. wenigstens einfach substituiertem Benzyl substituiert sein können, wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂ -NO₂, -CN, -CF₃, -CHF₂, - CH₂F, C₁₋₅-Alkyl und C₁₋₅-Alkoxy substituiert sein kann;
wobei die vorstehend genannten cycloaliphatischen Reste mit Substituenten unabhängig voneinander ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, - NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein können, wobei der jeweilige Furanyl-, Thiophenyl-, Pyridinyl-, Phenyl- oder Benzyl-Substituent selbst unsubstituiert oder mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy substituiert sein kann.

5. Verbindungen gemäß einem oder mehreren der Ansprüche 1 bis 4, **dadurch gekennzeichnet, daß** der Rest R⁵ für einen linearen oder verzweigten, gesättigten oder ungesättigten, unsubstituierten oder wenigstens einfach substituierten aliphatischen C₁₋₁₀-Rest, wobei die Substituenten unabhängig voneinander ausgewählt werden können aus der Gruppe bestehend aus Halogen, Hydroxy, -CN, -CF₃, -CHF₂, -CH₂F, C₁₋₆-Alkoxy und ggf. wenigstens einfach substituiertem Phenyl, dessen Substituenten jeweils unabhängig voneinander ausgewählt werden können aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, - CHF₂, -CH₂F.
C₁₋₅-Alkyl und C₁₋₅-Alkoxy, für einen gesättigten oder ungesättigten, unsubstituierten oder wenigstens einfach substituierten, ggf. wenigstens ein Heteroatom als Ringglied aufweisenden 3-, 4-, 5-, 6-, 7-, 8- oder 9-gliedrigen cycloaliphatischen Rest, der über eine lineare oder verzweigte C₁₋₃-Alkylen-Gruppe gebunden sein kann, oder für einen unsubstituierten oder wenigstens einfach substituierten, über eine lineare oder verzweigte C₁₋₃-Alkylen-Gruppe gebundenen, 5- bis 14-gliedrigen Aryl-Rest steht,
bevorzugt für einen linearen oder verzweigten C₁₋₅-Alkyl-Rest, für einen gesättigten oder ungesättigten 4-, 5-, 6-, 7-, 8- oder 9-gliedrigen cycloaliphatischen Rest, der über eine lineare oder verzweigte C₁₋₃-Alkylen-Gruppe gebunden sein kann, oder für einen unsubstituierten oder wenigstens einfach substituierten, über eine lineare oder verzweigte C₁₋₃-Alkylen-Gruppe gebundenen Phenyl-Rest steht,
besonders bevorzugt für einen Alkyl-Rest ausgewählt aus der Gruppe bestehend aus Methyl, Ethyl, Propyl, iso-Propyl, *n*-Butyl, iso-Butyl, sec-Butyl und *tert*-Butyl oder für einen Benzyl-Rest steht;
wobei die vorstehend genannten Aryl-Reste mit Substituenten unabhängig voneinander ausgewählt werden aus der Gruppe bestehend aus C₁₋₅-Alkyl, - C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein können, wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂ -NO₂, -CN, -CF₃, -CHF₂, - CH₂F, C₁₋₅-Alkyl und C₁₋₅-Alkoxy substituiert sein kann;
wobei die vorstehend genannten cycloaliphatischen Reste mit Substituenten unabhängig voneinander ausgewählt aus der Gruppe bestehend aus C₁₋₅-Alkyl, -C(=O)O-C₁₋₅-Alkyl, -O-C₁₋₅-Alkyl, Cl, F, Br, I, -C(=O)-C₁₋₅-Alkyl, -NH₂, - NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein können, wobei der jeweilige Furanyl-, Thiophenyl-, Pyridinyl-, Phenyl- oder Benzyl-Substituent selbst unsubstituiert oder mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy substituiert sein kann.

6. Verbindungen gemäß einem oder mehreren der Ansprüche 1 bis 5, **dadurch gekennzeichnet, daß** die Reste R⁴ und R⁵ zusammen mit dem sie verbindenden Stickstoffatom als Ringglied einen gesättigten oder ungesättigten, ggf. wenigstens ein weiteres Heteroatom als Ringglied aufweisenden 4-, 5-, 6-, 7-, 8- oder 9-gliedrigen cycloaliphatischen Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann,
bevorzugt zusammen mit dem sie verbindenden Stickstoffatom als Ringglied einen gesättigten oder ungesättigten, ggf. wenigstens ein weiteres Heteroatom ausgewählt aus der Gruppe bestehend aus Stickstoff, Sauerstoff und Schwefel als Ringglied aufweisenden 4-, 5-, 6-, 7-, 8- oder 9-gliedrigen cycloaliphatischen Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann,
besonders bevorzugt zusammen mit dem sie verbindenden Stickstoffatom als Ringglied einen Imidazolidinyl-, Aziridinyl-, Azetidinyl-, Pyrrolidinyl-, Piperidinyl-, Azepanyl-, Azokanyl-, Piperazinyl-, Morpholinyl- oder Thiomorpholinyl-Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituierten Pyridinyl, ggf. wenigstens einfach substituiertem Phenyl und ggf. wenigstens einfach substituiertem Benzyl substituiert sein kann;
wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, - CH₂F, C₁₋₅-Alkyl und C₁₋₅-Alkoxy substituiert sein kann.

7. Verbindungen gemäß einem oder mehreren der Ansprüche 1-6, **dadurch gekennzeichnet, daß**
die Reste R¹ und R² zusammen mit dem sie verbindenden Stickstoffatom als Ringglied einen gesättigten oder ungesättigten, ggf. wenigstens ein weiteres Heteroatom ausgewählt aus der Gruppe bestehend aus Stickstoff, Sauerstoff und Schwefel als Ringglied aufweisenden 4-, 5-, 6-, 7-, 8- oder 9-gliedrigen cycloaliphatischen Rest bilden, der einfach oder mehrfach, gleich oder verschieden mit einem Substituenten ausgewählt aus der Gruppe bestehend aus C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf. wenigstens einfach substituiertem Benzyl substituiert sein kann; wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, - CH₂F, C₁₋₅-Alkyl und C₁₋₅-Alkoxy substituiert sein kann;
der Rest R³ für einen linearen oder verzweigten, unsubstituierten oder wenigstens einfach substituierten C₁₋₅-Alkyl-Rest steht, wobei die Substituenten unabhängig voneinander ausgewählt werden können aus der Gruppe bestehend aus Halogen, Hydroxy, -CN, -CF₃, -CHF₂, -CH₂F, C₁₋₆-Alkoxy und ggf. wenigstens einfach substituiertem Phenyl, dessen Substituenten jeweils unabhängig voneinander ausgewählt werden können aus der Gruppe bestehend aus Hydroxy, F, Cl, Br, I, -NH₂, -NH-C₁₋₅-Alkyl, - N(C₁₋₅-Alkyl)₂, -NO₂, -CN, -CF₃, -CHF₂, -CH₂F, C₁₋₅-Alkyl und C₁₋₅-Alkoxy,
der Rest R⁴ für einen Wasserstoff-Rest, für einen linearen oder verzweigten C₁₋₅-Alkyl-Rest, für einen gesättigten oder ungesättigten 4-, 5-, 6-, 7-, 8- oder 9-gliedrigen cycloaliphatischen Rest, der über eine lineare oder verzweigte C₁₋₃-Alkylen-Gruppe gebunden sein kann, oder für einen unsubstituierten oder wenigstens einfach substituierten, über eine lineare oder verzweigte C₁₋₃-Alkylen-Gruppe gebundenen Phenyl-Rest steht,
der Rest R⁵ für einen linearen oder verzweigten C₁₋₅-Alkyl-Rest, für einen gesättigten oder ungesättigten 4-, 5-, 6-, 7-, 8- oder 9-gliedrigen cycloaliphatischen Rest, der über eine lineare oder verzweigte C₁₋₃-Alkylen-Gruppe gebunden sein kann, oder für einen unsubstituierten oder wenigstens einfach substituierten, über eine lineare oder verzweigte G₁₋₃-Alkylen-Gruppe gebundenen Phenyl-Rest steht, oder
die Reste R⁴ und R⁵ zusammen mit dem sie verbindenden Stickstoffatom als Ringglied einen gesättigten oder ungesättigten, ggf, wenigstens ein weiteres Heteroatom ausgewählt aus der Gruppe bestehend aus Stickstoff, Sauerstoff und Schwefel als Ringglied aufweisenden 4-, 5-, 6-, 7-, 8- oder 9-gliedrigen cycloaliphatischen Rest bilden, der einfach oder mehrfach, gleich oder verschieden mit einem Substituenten ausgewählt aus der Gruppe bestehend aus C₁₋₅-Alkyl, -C(=O)-Q-C₁₋₅-Alkyl, -C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf, wenigstens einfach substituiertem Phenyl und ggf. wenigstens einfach substituiertem Benzyl substituiert sein kann; wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂, -NO₂, -CN, -CF₃, -CHF₂, -CH₂F, C₁₋₅-Alkyl und C₁₋₅-Alkoxy substituiert sein kann;
wobei die vorstehen genannten Aryl-Reste mit Substituenten unabhängig voneinander ausgewählt werden aus der Gruppe bestehend aus C₁₋₅-Alkyl, - C(=O)-O-C₁₋₅-Alkyl, -O-C₁₋₅-Alkyl, Cl, F, Br, I, -C(=O)-C₁₋₅-Alkyl, -NH₂, -NH-C₁₋₅-Alkyl, -N(C₁₋₅-Alkyl)₂, ggf. wenigstens einfach substituiertem Furanyl, ggf. wenigstens einfach substituiertem Thiophenyl, ggf. wenigstens einfach substituiertem Pyridinyl, ggf. wenigstens einfach substituiertem Phenyl und ggf, wenigstens einfach substituiertem Benzyl substituiert sein können, wobei der jeweilige Furanyl-, Thiophenyl-, Pyridinyl-, Phenyl- oder Benzyl-Substituent selbst unsubstituiert oder einfach oder mehrfach mit Substituenten unabhängig voneinander ausgewählt aus der Gruppe bestehend aus Hydroxy, F, Cl, Br, I, -NH₂, -NH-C₁₋₅-Alkyl, -N(C₁₋₅-Alkyl)₂ -NO₂, -CN, -CF₃, -CHF₂, - CH₂F, C₁₋₅-Alkyl und C₁₋₅-Alkoxy substituiert sein kann.

8. Verbindungen gemäß einem oder mehreren der Ansprüche 1 bis 7, **dadurch gekennzeichnet, daß**
R¹ und R² zusammen mit dem sie verbindenden Stickstoffatom als Ringglied einen Imidazolidinyl-, Aziridinyl-, Azetidinyl-, Pyrroliding-, Piperidinyl-, Azepanyl-, Azokanyl-, Piperazinyl-, Morpholinyl- oder Thiomorpholinyl-Rest bilden, der mit einem Substituenten ausgewählt aus der Gruppe bestehend aus Methyl, Ethyl, *n*-Propyl, iso-Propyl, *n*-Butyl, iso-Butyl, sec-Butyl, tert.-Butyl, Phenyl und Benzyl substituiert sein kann,
R³ für einen Alkyl-Rest ausgewählt aus der Gruppe bestehend aus Methyl, Ethyl, n-Propyl, iso-Propyl, *n*-Butyl, iso-Butyl, sec-Butyl und *tert*-Butyl steht,
R⁴ für einen Wasserstoff-Rest, für einen Alkyl-Rest ausgewählt aus der Gruppe bestehend aus Methyl, Ethyl, n-Propyl, iso-Propyl, *n*-Butyl, iso-Butyl, sec-Butyl, *tert*-Butyl, n-Pentyl, iso-Pentyl und neo-Pentyl, oder für einen Benzyl-Rest steht,
R⁵ für einen Alkyl-Rest ausgewählt aus der Gruppe bestehend aus Methyl, Ethyl, n-Propyl, iso-Propyl, *n*-Butyl, iso-Butyl, sec-Butyl, *tert*-Butyl, *n*-Pentyl, iso-Pentyl und neo-Pentyl, oder für einen Benzyl-Rest steht,
oder
R⁴ und R⁵ zusammen mit dem sie verbindenden Stickstoffatom als Ringglied einen Imidazolidinyl-, Aziridinyl-, Azetidinyl-, Pyrrolidinyl-, Piperidinyl-, Azepanyl-, Azokanyl-, Piperazinyl-, Morpholinyl- oder Thiomorpholinyl-Rest bilden, der mit einem Substituenten ausgewählt aus der Gruppe bestehend aus Methyl, Ethyl, n-Propyl, iso-Propyl, *n*-Butyl, iso-Butyl, sec-Butyl, tert-Butyl, Phenyl und Benzyl substituiert sein kann.

9. Verbindungen gemäß einem oder mehreren der Ansprüche 1 bis 8 ausgewählt aus der Gruppe bestehend aus
Diethyl-(1-methyl-5-piperidin-1-ylmethyl-1H-pyrrol-2-ylmethyl)-amin,
1-(1-Methyl-5-piperidin-1-ylmethyl-1H-pyrrol-2-ylmethyl)-4-methylpiperidin,
4-[1-Methyl-5-(4-phenyl-piperidin-1-ylmethyl)-1H-pyrrol-2-ylmethyl]-morpholin,
1-(1-Methyl-5-piperidin-1-ylmethyl-1H-pyrrol-2-ylmethyl)-4-phenyl-piperazin,
1-[5-(4-Benzyl-piperidin-1-ylmethyl)-1-methyl-1H-pyrrol-2-ylmethyl]-azepan,
Benzyl-[5-(4-benzyl-piperidin-1-ylmethyl)-1-methyl-1H-pyrrol-2-ylmethyl]-methylamin,
4-[5-(4-Benzyl-piperidin-1-ylmethyl)-1-methyl-1H-pyrrol-2-ylmethyl]-morpholin,
(5-Azepan-1-ylmethyl-1-methyl-1H-pyrrol-2-ylmethyl)-benzyl-methyl-amin,
Benzyl-methyl-[1-methyl-5-(4-phenyl-piperidin-1-ylmethyl)-1H-pyrrol-2-ylmethyl]-amin und
Benzyl-methyl-[1-methyl-5-(4-phenyl-piperidin-1-ylmethyl)-1H-pyrrol-2-ylmethyl]-amin,
jeweils ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

10. Verfahren zur Herstellung von 2,5-Diaminomethyl-1H-pyrrolverbindungen der allgemeinen Formel I gemäß einem oder mehreren der Ansprüche 1 bis 9, **dadurch gekennzeichnet, daß** eine Verbindung der allgemeinen Formel II, 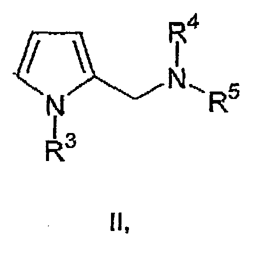 worin die Reste R³, R⁴ und R⁵ die Bedeutung gemäß einem oder mehreren der Ansprüche 1 bis 9 haben, mit einem Iminiumsalz der allgemeinen Formel III, 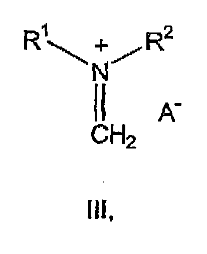 worin die Reste R¹ und R² die Bedeutung gemäß einem oder mehreren der Ansprüche 1 bis 9 haben, und A⁻ für ein geeignetes Anion, vorzugsweise Cl⁻, AlCl₄⁻, Br⁻, I⁻ oder CF₃-SO₃⁻ (Triflat-Anion) steht, umgesetzt und die so erhaltene Verbindung ggf. gereinigt und ggf, isoliert wird.

11. Arzneimittel enthaltend wenigstens eine Verbindung gemäß einem oder mehreren der Ansprüche 1 bis 9 einschließlich der ausgenommenen Verbindungen sowie ggf. einen oder mehrere physiologisch verträgliche Hilfsstoffe.

12. Arzneimittel gemäß Anspruch 11 zur Regulation, vorzugsweise Inhibierung, des 5-Hydroxytryptamin-(5-HT)-Uptakes.

13. Arzneimittel gemäß Anspruch 11 zur Regulation, vorzugsweise Inhibierung, des Noradrenalin(NA)-Uptakes.

14. Arzneimittel gemäß Anspruch 11 zur Opioid-Rezeptor-Regulation, vorzugsweise zur µ-Opiold-Rezeptor-Regulation.

15. Arzneimittel gemäß einem oder mehreren der Ansprüche 11 bis 14 zur Prophylaxe und/oder Behandlung von Störungen oder Krankheiten, die zumindest teilweise durch 5-Hydroxytryptamin-(5-HT)-Rezeptoren und/oder Noradrenalin(NA)-Rezeptoren und/oder Opioid-Rezeptoren, insbesondere µ-Opioid-Rezeptoren vermittelt werden.

16. Arzneimittel gemäß einem oder mehreren der Ansprüche 11 bis 15 zur Behandlung von Schmerzen.

17. Arzneimittel gemäß Anspruch 16 zur Behandlung von Schmerzen ausgewählt aus der Gruppe bestehend aus akuten Schmerzen, chronischen Schmerzen und neuropathischen Schmerzen.

18. Arzneimittel gemäß einem oder mehreren der Ansprüche 11 bis 15 zur Prophylaxe und/oder Behandlung von Entzugserscheinungen, Gedächtnisstörungen, neurodegenerativen Erkrankungen, vorzugsweise ausgewählt aus der Gruppe bestehend aus Morbus Parkinson, Morbus Huntington, Morbus Alzheimer und Multipler Sklerose, Epilepsie, cardiovaskulären Störungen, Wasserspeicher-Krankheiten, intestinaler Motilität (Diarrhoe), Harninkontinenz, Anorexie, Tinnitus, Pruritus, Depressionen, sexueller Dysfunktionen, vorzugsweise erektiler Dysfunktion oder Atemwegserkrankungen, Störungen der Nahrungsmittelaufnahme, vorzugsweise ausgewählt aus der Gruppe bestehend aus Fettsucht, Bulimie, Anorexie, Kachexie und Typ II Diabetes (nicht-insulinabhängiger Diabetes), oder zur Anxiolyse, zur Diurese, zur Unterdrückung des Miktionsreflexes, zur Reduzierung des Suchtpotentials von Opioiden, vorzugsweise Morphin, zur Modulation der Bewegungsaktivität, zur Beeinflussung des cardiovaskulären Systems, vorzugsweise zur Vasodilatation der Arterien, oder zur Regulation des Elektrolyt-Haushaltes.

19. Verwendung wenigstens einer Verbindung gemäß einem oder mehreren der Ansprüche 1 bis 9 einschließlich der ausgenommenen Verbindungen zur Herstellung eines Arzneimittels zur Regulation des 5-Hydroxytryptamin-(5-HT)-Uptakes, vorzugsweise zur Inhibierung des 5-Hydroxytryptamin-(5-HT)-Uptakes.

20. Verwendung wenigstens einer Verbindung gemäß einem oder mehreren der Ansprüche 1 bis 9 einschließlich der ausgenommenen Verbindungen zur Herstellung eines Arzneimittels zur Regulation des Noradrenalin(NA)-Uptakes, vorzugsweise zur Inhibierung des Noradrenalin(NA)-Uptakes.

21. Verwendung wenigstens einer Verbindung gemäß einem oder mehreren der Ansprüche 1 bis 9 einschließlich der ausgenommenen Verbindungen zur Herstellung eines Arzneimittels zur Opioid-Rezeptor-Regulation, vorzugsweise zur µ-Opioid-Rezeptor-Regulation.

22. Verwendung wenigstens einer Verbindung gemäß einem oder mehreren der Ansprüche 1 bis 9 einschließlich der ausgenommenen Verbindungen zur Herstellung eines Arzneimittels zur Prophylaxe und/oder Behandlung von Störungen oder Krankheiten, die zumindest teilweise durch 5-Hydroxytryptamin-(5-HT)-Rezeptoren und/oder Noradrenalin(NA)-Rezeptoren und/oder Opioid-Rezeptoren, insbesondere µ-Opioid-Rezeptoren, vermittelt werden.

23. Verwendung wenigstens einer Verbindung gemäß einem oder mehreren der Ansprüche 1 bis 9 einschließlich der ausgenommenen Verbindungen zur Herstellung eines Arzneimittels zur Behandlung von Schmerzen, vorzugsweise von Schmerzen ausgewählt aus der Gruppe bestehend aus akuten Schmerzen, chronischen Schmerzen und neuropathischen Schmerzen,

24. Verwendung wenigstens einer Verbindung gemäß einem oder mehreren der Ansprüche 1 bis 9 einschließlich der ausgenommenen Verbindungen zur Herstellung eines Arzneimittels zur Prophylaxe und/oder Behandlung von Entzugserscheinungen, Gedächtnisstörungen, neurodegenerativen Erkrankungen, vorzugsweise ausgewählt aus der Gruppe bestehend aus Morbus Parkinson, Morbus Huntington, Morbus Alzheimer und Multipler Sklerose, Epilepsie, cardiovaskulären Störungen, Wasserspeicher-Krankheiten, intestinaler Motilität (Diarrhoe), Harninkontinenz, Anorexie, Tinnitus, Pruritus, Depressionen, sexueller Dysfunktionen, vorzugsweise erektiler Dysfunktion oder Atemwegserkrankungen, Störungen der Nahrungsmittelaufnahme, vorzugsweise ausgewählt aus der Gruppe bestehend aus Fettsucht, Bulimie, Anorexie, Kachexie und Typ II Diabetes (nicht-insulinabhängiger Diabetes), oder zur Anxiolyse, zur Diurese, zur Unterdrückung des Miktionsreflexes, zur Reduzierung des Suchtpotentials von Opioiden, vorzugsweise Morphin, zur Modulation der Bewegungsaktivität, zur Beeinflussung des cardiovaskulären Systems, vorzugsweise zur Vasodilatation der Arterien, oder zur Regulation des Elektrolyt-Haushaltes.

## Claims

1. Substituted 2,5-diaminomethyl-1H-pyrroles of the general formula I, 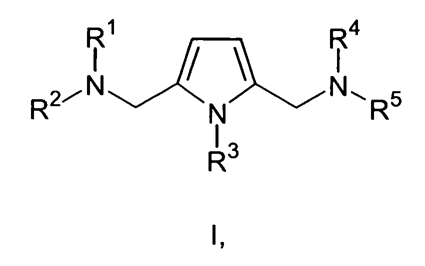 in which
R¹ and R² together with the nitrogen atom joining them together as a ring member form a saturated or unsaturated cycloaliphatic residue optionally comprising at least one further heteroatom as a ring member, which cycloaliphatic residue may be identically or differently mono- or polysubstituted with a substituent selected from the group consisting of C₁₋₅ alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl; wherein the particular furanyl, thiophenyl, pyridinyl, phenyl or benzyl substituent may itself be unsubstituted or mono- or polysubstituted with substituents mutually independently selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy;
R³ denotes a linear or branched, unsaturated or saturated, unsubstituted or at least monosubstituted aliphatic residue,
R⁴ denotes a hydrogen residue, a linear or branched, unsubstituted or at least monosubstituted, unsaturated or saturated aliphatic residue, an unsaturated or saturated, unsubstituted or at least monosubstituted, cycloaliphatic residue optionally comprising at least one heteroatom as a ring member, which cycloaliphatic residue may be attached via a linear or branched alkylene group, or an unsubstituted or at least monosubstituted aryl residue attached via a linear or branched alkylene group,
R⁵ denotes a linear or branched, unsubstituted or at least monosubstituted, unsaturated or saturated aliphatic residue, an unsaturated or saturated, unsubstituted or at least monosubstituted cycloaliphatic residue optionally comprising at least one heteroatom as a ring member, which cycloaliphatic residue may be attached via a linear or branched alkylene group, or an unsubstituted or at least monosubstituted aryl residue attached via a linear or branched C₁₋₃ alkylene group,
or
R⁴ and R⁵ together with the nitrogen atom joining them together as a ring member form a saturated or unsaturated cycloaliphatic residue optionally comprising at least one further heteroatom as a ring member, which cycloaliphatic residue may be identically or differently mono- or polysubstituted with a substituent selected from the group consisting of C₁₋₅ alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl; wherein the particular furanyl, thiophenyl, pyridinyl, phenyl or benzyl substituent may itself be unsubstituted or mono- or polysubstituted with substituents mutually independently selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy;
wherein the above-stated aliphatic residues may be substituted with substituents mutually independently selected from the group consisting of halogen, hydroxy, -CN, -CF₃, -CHF₂, -CH₂F, C₁₋₆-alkoxy and optionally at least monosubstituted phenyl, the substituents of which may in each case mutually independently be selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy;
wherein the above-stated aryl residues may be substituted with substituents mutually independently selected from the group consisting of C₁₋₅-alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl, wherein the particular furanyl, thiophenyl, pyridinyl, phenyl or benzyl substituent may itself be unsubstituted or mono- or polysubstituted with substituents mutually independently selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy;
wherein the above-stated cycloaliphatic residues in the position of residues R⁴ and R⁵ may be substituted with substituents mutually independently selected from the group consisting of C₁₋₅-alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl, wherein the particular furanyl, thiophenyl, pyridinyl, phenyl or benzyl substituent may itself be unsubstituted or substituted with substituents mutually independently selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy;
in each case optionally in the form of the pure stereoisomers thereof, in particular enantiomers or diastereomers, the racemates thereof or in the form of mixtures of the stereoisomers, in particular the enantiomers and/or diastereomers, in any desired mixing ratio, or in each case in the form of corresponding salts or in each case in the form of corresponding solvates, with the proviso that compounds of the general formula I, in which
residues R¹ and R² and residues R⁴ and R⁵ in each case together with the nitrogen atom joining them together as a ring member form the same residue selected from the group consisting of pyrrolidinyl, morpholinyl and piperidinyl, and residue R³ in each case denotes a methyl group, and
residues R¹ and R² together with the nitrogen atom joining them together as a ring member form a piperidinyl residue and residues R³ to R⁵ in each case denote a methyl group,
are excepted.

2. Compounds according to claim 1, **characterised in that** residues R¹ and R² together with the nitrogen atom joining them together as a ring member form a saturated or unsaturated 4-, 5-, 6-, 7-, 8- or 9-membered cycloaliphatic residue optionally comprising at least one further heteroatom as a ring member, which cycloaliphatic residue may be identically or differently mono- or polysubstituted with a substituent selected from the group consisting of C₁₋₅ alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl,
together with the nitrogen atom joining them together as a ring member preferably form a saturated or unsaturated 4-, 5-, 6-, 7-, 8- or 9-membered cycloaliphatic residue optionally comprising at least one further heteroatom selected from the group consisting of nitrogen, oxygen and sulfur as a ring member, which cycloaliphatic residue may be identically or differently mono- or polysubstituted with a substituent selected from the group consisting of C₁₋₅ alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl,
together with the nitrogen atom joining them together as a ring member particularly preferably form an imidazolidinyl, aziridinyl, azetidinyl, pyrrolidinyl, piperidinyl, azepanyl, azocanyl, piperazinyl, morpholinyl or thiomorpholinyl residue, which residue may be identically or differently mono- or polysubstituted with a substituent selected from the group consisting of C₁₋₅ alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl,
wherein the particular furanyl, thiophenyl, pyridinyl, phenyl or benzyl substituent may itself be unsubstituted or mono- or polysubstituted with substituents mutually independently selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy.

3. Compounds according to claim 1 or claim 2, **characterised in that** residue R³ denotes a linear or branched, saturated or unsaturated, unsubstituted or at least monosubstituted aliphatic C₁₋₁₀ residue, wherein the substituents may mutually independently be selected from the group consisting of halogen, hydroxy, -CN, -CF₃, -CHF₂, -CH₂F, C₁₋₆-alkoxy and optionally at least monosubstituted phenyl, the substituents of which may in each case mutually independently be selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy,
preferably denotes a linear or branched, unsubstituted or at least monosubstituted C₁₋₅ alkyl residue, wherein the substituents may mutually independently be selected from the group consisting of halogen, hydroxy, -CN, -CF₃, -CHF₂, -CH₂F, C₁₋₆-alkoxy and optionally at least monosubstituted phenyl, the substituents of which may in each case mutually independently be selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy,
particularly preferably denotes an alkyl residue selected from the group consisting of methyl, ethyl, n-propyl, iso-propyl, *n*-butyl, iso-butyl, sec-butyl and *tert.*-butyl.

4. Compounds according to one or more of claims 1 to 3, **characterised in that** residue R⁴ denotes a hydrogen residue, a linear or branched, saturated or unsaturated, unsubstituted or at least monosubstituted aliphatic C₁₋₁₀ residue, wherein the substituents may mutually independently be selected from the group consisting of halogen, hydroxy, -CN, -CF₃, -CHF₂, -CH₂F, C₁₋₆-alkoxy and optionally at least monosubstituted phenyl, the substituents of which may in each case mutually independently be selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy; a saturated or unsaturated, unsubstituted or at least monosubstituted 3-, 4-, 5-, 6-, 7-, 8- or 9-membered cycloaliphatic residue optionally comprising at least one heteroatom as a ring member, which cycloaliphatic residue may be attached via a linear or branched C₁₋₃ alkylene group, or an unsubstituted or at least monosubstituted 5- to 14-membered aryl residue attached via a linear or branched C₁₋₃ alkylene group,
preferably denotes a hydrogen residue, a linear or branched C₁₋₅ alkyl residue, a saturated or unsaturated 4-, 5-, 6-, 7-, 8- or 9-membered cycloaliphatic residue, which may be attached via a linear or branched C₁₋₃ alkylene group, or an unsubstituted or at least monosubstituted phenyl residue attached via a linear or branched C₁₋₃ alkylene group,
particularly preferably denotes a hydrogen residue, an alkyl residue selected from the group consisting of methyl, ethyl, propyl, iso-propyl, *n*-butyl, iso-butyl, sec-butyl and *tert*.-butyl or a benzyl residue;
wherein the above-stated aryl residues may be substituted with substituents mutually independently selected from the group consisting of C₁₋₅-alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl, wherein the particular furanyl, thiophenyl, pyridinyl, phenyl or benzyl substituent may itself be unsubstituted or mono- or polysubstituted with substituents mutually independently selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy;
wherein the above-stated cycloaliphatic residues may be substituted with substituents mutually independently selected from the group consisting of C₁₋₅-alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl, wherein the particular furanyl, thiophenyl, pyridinyl, phenyl or benzyl substituent may itself be unsubstituted or substituted with substituents mutually independently selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy.

5. Compounds according to one or more of claims 1 to 4, **characterised in that** residue R⁵ denotes a linear or branched, saturated or unsaturated, unsubstituted or at least monosubstituted aliphatic C₁₋₁₀ residue, wherein the substituents may mutually independently be selected from the group consisting of halogen, hydroxy, -CN, -CF₃, -CHF₂, -CH₂F, C₁₋₆-alkoxy and optionally at least monosubstituted phenyl, the substituents of which may in each case mutually independently be selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy; a saturated or unsaturated, unsubstituted or at least monosubstituted 3-, 4-, 5-, 6-, 7-, 8- or 9-membered cycloaliphatic residue optionally comprising at least one heteroatom as a ring member, which cycloaliphatic residue may be attached via a linear or branched C₁₋₃ alkylene group, or an unsubstituted or at least monosubstituted 5- to 14-membered aryl residue attached via a linear or branched C₁₋₃ alkylene group,
preferably denotes a linear or branched C₁₋₅ alkyl residue, a saturated or unsaturated 4-, 5-, 6-, 7-, 8- or 9-membered cycloaliphatic residue, which may be attached via a linear or branched C₁₋₃ alkylene group, or denotes an unsubstituted or at least monosubstituted phenyl residue attached via a linear or branched C₁₋₃ alkylene group,
particularly preferably denotes an alkyl residue selected from the group consisting of methyl, ethyl, propyl, iso-propyl, n-butyl, iso-butyl, sec-butyl and tert.-butyl or a benzyl residue,
wherein the above-stated aryl residues may be substituted with substituents mutually independently selected from the group consisting of C₁₋₅-alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl, wherein the particular furanyl, thiophenyl, pyridinyl, phenyl or benzyl substituent may itself be unsubstituted or mono- or polysubstituted with substituents mutually independently selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy;
wherein the above-stated cycloaliphatic residues may be substituted with substituents mutually independently selected from the group consisting of C₁₋₅-alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl, wherein the particular furanyl, thiophenyl, pyridinyl, phenyl or benzyl substituent may itself be unsubstituted or substituted with substituents mutually independently selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy.

6. Compounds according to one or more of claims 1 to 5, **characterised in** residues R⁴ and R⁵ together with the nitrogen atom joining them together as a ring member form a saturated or unsaturated 4-, 5-, 6-, 7-, 8- or 9-membered cycloaliphatic residue optionally comprising at least one further heteroatom as a ring member, which cycloaliphatic residue may be identically or differently mono- or polysubstituted with a substituent selected from the group consisting of C₁₋₅ alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl,
together with the nitrogen atom joining them together as a ring member preferably form a saturated or unsaturated 4-, 5-, 6-, 7-, 8- or 9-membered cycloaliphatic residue optionally comprising at least one further heteroatom selected from the group consisting of nitrogen, oxygen and sulfur as a ring member, which cycloaliphatic residue may be identically or differently mono- or polysubstituted with a substituent selected from the group consisting of C₁₋₅ alkyl, -C(=O)-O-C₁₋₅-alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl,
together with the nitrogen atom joining them together as a ring member particularly preferably form an imidazolidinyl, aziridinyl, azetidinyl, pyrrolidinyl, piperidinyl, azepanyl, azocanyl, piperazinyl, morpholinyl or thiomorpholinyl residue, which residue may be identically or differently mono- or polysubstituted with a substituent selected from the group consisting of C₁₋₅ alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and
optionally at least monosubstituted benzyl;
wherein the particular furanyl, thiophenyl, pyridinyl, phenyl or benzyl substituent may itself be unsubstituted or mono- or polysubstituted with substituents mutually independently selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy.

7. Compounds according to one or more of claims 1-6, **characterised in that**
residues R¹ and R² together with the nitrogen atom joining them together as a ring member form a saturated or unsaturated 4-, 5-, 6-, 7-, 8- or 9-membered cycloaliphatic residue optionally comprising at least one further heteroatom selected from the group consisting of nitrogen, oxygen and sulfur as a ring member, which cycloaliphatic residue may be identically or differently mono- or polysubstituted with a substituent selected from the group consisting of C₁₋₅ alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl; wherein the particular furanyl, thiophenyl, pyridinyl, phenyl or benzyl substituent may itself be unsubstituted or mono- or polysubstituted with substituents mutually independently selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy;
residue R³ denotes a linear or branched, unsubstituted or at least monosubstituted C₁₋₅ alkyl residue, wherein the substituents may mutually independently be selected from the group consisting of halogen, hydroxy, -CN, -CF₃, -CHF₂, -CH₂F, C₁₋₆-alkoxy and optionally at least monosubstituted phenyl, the substituents of which may in each case mutually independently be selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy,
residue R⁴ denotes a hydrogen residue, a linear or branched C₁₋₅ alkyl residue, a saturated or unsaturated 4-, 5-, 6-, 7-, 8- or 9-membered cycloaliphatic residue, which may be attached via a linear or branched C₁₋₃ alkylene group, or an unsubstituted or at least monosubstituted phenyl residue attached via a linear or branched C₁₋₃ alkylene group,
residue R⁵ denotes a linear or branched C₁₋₅ alkyl residue, a saturated or unsaturated 4-, 5-, 6-, 7-, 8- or 9-membered cycloaliphatic residue, which may be attached via a linear or branched C₁₋₃ alkylene group, or denotes an unsubstituted or at least monosubstituted phenyl residue attached via a linear or branched C₁₋₃ alkylene group, or
residues R⁴ and R⁵ together with the nitrogen atom joining them together as a ring member form a saturated or unsaturated 4-, 5-, 6-, 7-, 8- or 9-membered cycloaliphatic residue optionally comprising at least one further heteroatom selected from the group consisting of nitrogen, oxygen and sulfur as a ring member, which cycloaliphatic residue may be identically or differently mono- or polysubstituted with a substituent selected from the group consisting of C₁₋₅ alkyl, -C(=O)-O-C₁₋₅-alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl; wherein the particular furanyl, thiophenyl, pyridinyl, phenyl or benzyl substituent may itself be unsubstituted or mono- or polysubstituted with substituents mutually independently selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy;
wherein the above-stated aryl residues may be substituted with substituents mutually independently selected from the group consisting of C₁₋₅-alkyl, -C(=O)-O-C₁₋₅-alkyl, -O-C₁₋₅-alkyl, Cl, F, Br, I, -C(=O)-C₁₋₅-alkyl, -NH₂, -NH-C₁₋₅-alkyl, -N(C₁₋₅-alkyl)₂, optionally at least monosubstituted furanyl, optionally at least monosubstituted thiophenyl, optionally at least monosubstituted pyridinyl, optionally at least monosubstituted phenyl and optionally at least monosubstituted benzyl, wherein the particular furanyl, thiophenyl, pyridinyl, phenyl or benzyl substituent may itself be unsubstituted or mono- or polysubstituted with substituents mutually independently selected from the group consisting of hydroxy, F, Cl, Br, I, -NH₂, -NH-C₁₋₅-alkyl, -N(C₁₋₅-alkyl)₂, -NO₂, -CN, -CF₃, -CHF₂, -CH₂F, C₁₋₅-alkyl and C₁₋₅ alkoxy.

8. Compounds according to one or more of claims 1 to 7, **characterised in that**
R¹ and R² together with the nitrogen atom joining them together as a ring member form an imidazolidinyl, aziridinyl, azetidinyl, pyrrolidinyl, piperidinyl, azepanyl, azocanyl, piperazinyl, morpholinyl or thiomorpholinyl residue, which may be substituted with a substituent selected from the group consisting of methyl, ethyl, n-propyl, iso-propyl, n-butyl, iso-butyl, sec-butyl, *tert*.-butyl, phenyl and benzyl,
R³ denotes an alkyl residue selected from the group consisting of methyl, ethyl, n-propyl, iso-propyl, *n*-butyl, iso-butyl, sec-butyl and *tert.*-butyl,
R⁴ denotes a hydrogen residue, an alkyl residue selected from the group consisting of methyl, ethyl, n-propyl, iso-propyl, *n*-butyl, iso-butyl, sec-butyl, *tert*.-butyl, n-pentyl, iso-pentyl and neopentyl, or a benzyl residue,
R⁵ denotes a residue selected from the group consisting of methyl, ethyl, n-propyl, iso-propyl, *n*-butyl, iso-butyl, sec-butyl, *tert*.-butyl, n-pentyl, iso-pentyl and neopentyl, or a benzyl residue,
or
R⁴ and R⁵ together with the nitrogen atom joining them together as a ring member form an imidazolidinyl, aziridinyl, azetidinyl, pyrrolidinyl, piperidinyl, azepanyl, azocanyl, piperazinyl, morpholinyl or thiomorpholinyl residue, which may be substituted with a substituent selected from the group consisting of methyl, ethyl, n-propyl, iso-propyl, *n*-butyl, iso-butyl, sec-butyl, *tert.*-butyl, phenyl and benzyl.

9. Compounds according to one or more of claims 1 to 8 selected from the group consisting of
dimethyl-(1-methyl-5-piperidin-1-ylmethyl-1H-pyrrol-2-ylmethyl)-amine,
1-(1-methyl-5-piperidin-1-ylmethyl-1H-pyrrol-2-ylmethyl)-4-methylpiperidine,
4-[1-methyl-5-(4-phenyl-piperidin-1-ylmethyl)-1H-pyrrol-2-ylmethyl]-morpholine,
1-(1-methyl-5-piperidin-1-ylmethyl-1H-pyrrol-2-ylmethyl)-4-phenyl-piperazine,
1-[5-(4-benzyl-piperidin-1-ylmethyl)-1-methyl-1H-pyrrol-2-ylmethyl]-azepane,
benzyl-[5-(4-benzyl-piperidin-1-ylmethyl)-1-methyl-1H-pyrrol-2-ylmethyl]-methylamine,
4-[5-(4-benzyl-piperidin-1-ylmethyl)-1-methyl-1H-pyrrol-2-ylmethyl]-morpholine,
(5-azepan-1-ylmethyl-1-methyl-1H-pyrrol-2-ylmethyl)-benzyl-methyl-amine,
benzyl-methyl-[1-methyl-5-(4-phenyl-piperidin-1-ylmethyl)-1H-pyrrol-2-ylmethyl]-amine and
benzyl-methyl-[1-methyl-5-(4-phenyl-piperidin-1-ylmethyl)-1H-pyrrol-2-ylmethyl]-amine,
in each case optionally in the form of the pure stereoisomers thereof, in particular enantiomers or diastereomers, the racemates thereof or in the form of mixtures of the stereoisomers, in particular the enantiomers and/or diastereomers, in any desired mixing ratio, or in each case in the form of corresponding salts or in each case in the form of corresponding solvates.

10. A method for the production of 2,5-diaminomethyl-1 H-pyrrole compounds of the general formula I according to one or more of claims 1 to 9, **characterised in that** a compound of the general formula II, 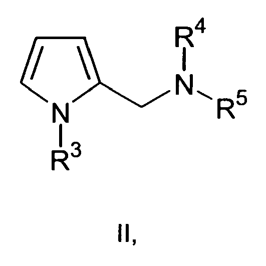 in which residues R³ to R⁴ and R⁵ have the meaning according to one or more of claims 1 to 9, is reacted with an iminium salt of the general formula III, 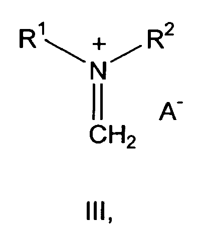 in which residues R¹ and R² have the meaning according to one or more of claims 1 to 9, and A⁻ denotes a suitable anion, preferably Cl⁻, AlCl₄⁻, Br⁻, I⁻ or CF₃-SO₃⁻ (triflate anion) and the resultant compound is optionally purified and optionally isolated.

11. A medicament containing at least one compound according to one or more of claims 1 to 9 including the excepted compounds and optionally one or more physiologically acceptable auxiliary substances.

12. A medicament according to claim 11 for regulating, preferably inhibiting, 5-hydroxytryptamine (5-HT) uptake.

13. A medicament according to claim 11 for regulating, preferably inhibiting, noradrenalin (NA) uptake.

14. A medicament according to claim 11 for opioid receptor regulation, preferably for µ opioid receptor regulation.

15. A medicament according to one or more of claims 11 to 14 for the prevention and/or treatment of disorders or diseases, which are at least partially mediated by 5-hydroxytryptamine (5-HT) receptors and/or noradrenalin (NA) receptors and/or opioid receptors, in particular µ opioid receptors.

16. A medicament according to one or more of claims 11 to 15 for the treatment of pain.

17. A medicament according to claim 16 for the treatment of pain selected from the group consisting of acute pain, chronic pain, and neuropathic pain.

18. A medicament according to one or more of claims 11 to 15 for the prevention and/or treatment of withdrawal symptoms, memory disorders, neurodegenerative diseases, preferably selected from the group consisting of Parkinson's disease, Huntington's chorea, Alzheimer's disease and multiple sclerosis, epilepsy, cardiovascular disorders, water retention conditions, intestinal motility (diarrhoea), urinary incontinence, anorexia, tinnitus, pruritus, depression, sexual dysfunction, preferably erectile dysfunction, or airways diseases, disorders of food intake, preferably selected from the group consisting of obesity, bulimia, anorexia, cachexia and type II diabetes (non-insulin-dependent diabetes), or for anxiolysis, for diuresis, for suppressing the urinary reflex, for reducing the addictive potential of opioids, preferably morphine, for modulating locomotor activity, for influencing the cardiovascular system, preferably for vasodilating the arteries, or for regulating the electrolyte balance.

19. Use of at least one compound according to one or more of claims 1 to 9 including the excepted compounds for the production of a medicament for regulating 5-hydroxytryptamine (5-HT) uptake, preferably for inhibiting 5-hydroxytryptamine (5-HT) uptake.

20. Use of at least one compound according to one or more of claims 1 to 9 including the excepted compounds for the production of a medicament for regulating noradrenalin (NA) uptake, preferably for inhibiting noradrenalin (NA) uptake.

21. Use of at least one compound according to one or more of claims 1 to 9 including the excepted compounds for the production of a medicament for opioid receptor regulation, preferably for µ opioid receptor regulation.

22. Use of at least one compound according to one or more of claims 1 to 9 including the excepted compounds for the production of a medicament for the prevention and/or treatment of disorders or diseases which are at least partially mediated by 5-hydroxytryptamine (5-HT) receptors and/or noradrenalin (NA) receptors and/or opioid receptors, in particular µ opioid receptors.

23. Use of at least one compound according to one or more of claims 1 to 9 including the excepted compounds for the production of a medicament for the treatment of pain, preferably of pain selected from the group consisting of acute pain, chronic pain, and neuropathic pain.

24. Use of at least one compound according to one or more of claims 1 to 9 including the excepted compounds for the production of a medicament for the prevention and/or treatment of withdrawal symptoms, memory disorders, neurodegenerative diseases, preferably selected from the group consisting of Parkinson's disease, Huntington's chorea, Alzheimer's disease and multiple sclerosis, epilepsy, cardiovascular disorders, water retention conditions, intestinal motility (diarrhoea), urinary incontinence, anorexia, tinnitus, pruritus, depression, sexual dysfunction, preferably erectile dysfunction, or airways diseases, disorders of food intake, preferably selected from the group consisting of obesity, bulimia, anorexia, cachexia and type II diabetes (non-insulin-dependent diabetes), or for anxiolysis, for diuresis, for suppression of the urinary reflex, for reducing the addictive potential of opioids, preferably morphine, for modulating locomotor activity, for influencing the cardiovascular system, preferably for vasodilating the arteries, or for regulating the electrolyte balance.

## Revendications

1. 2,5-diaminométhyl-1H-pyrroles substitués de formule générale I, 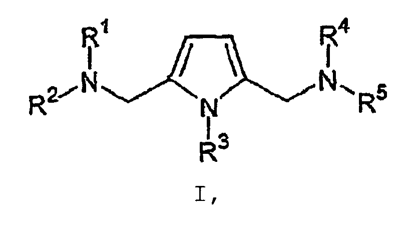 dans laquelle
R¹ et R² forment ensemble, avec l'atome d'azote qui les relie en tant que chaînon de cycle, un radical cycloaliphatique saturé ou insaturé, comportant éventuellement au moins un autre hétéroatome en tant que chaînon de cycle, qui peut être une ou plusieurs fois substitué, de façon identique ou différente, par un substituant choisi dans l'ensemble constitué par des groupes alkyle en C₁-C₅, -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N[alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué ; le substituant furannyle, thiophényle, pyridinyle, phényle ou benzyle respectif pouvant lui-même être non substitué ou une ou plusieurs fois substitué par des substituants choisis, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅ ;
R³ représente un radical aliphatique linéaire ou ramifié, saturé ou insaturé, non substitué ou au moins monosubstitué,
R⁴ représente un atome d'hydrogène, un radical aliphatique linéaire ou ramifié, saturé ou insaturé, non substitué ou au moins monosubstitué, un radical cycloaliphatique saturé ou insaturé, non substitué ou au moins monosubstitué, comportant éventuellement au moins un hétéroatome en tant que chaînon de cycle, qui peut être lié par un groupe alkylène linéaire ou ramifié, ou un radical aryle non substitué ou au moins monosubstitué, lié par un groupe alkylène linéaire ou ramifié,
R⁵ représente un radical aliphatique linéaire ou ramifié, saturé ou insaturé, non substitué ou au moins monosubstitué, un radical cycloaliphatique saturé ou insaturé, non substitué ou au moins monosubstitué, comportant éventuellement au moins un hétéroatome en tant que chaînon de cycle, qui peut être lié par un groupe alkylène linéaire ou ramifié, ou un radical aryle non substitué ou au moins monosubstitué, lié par un groupe alkylène en C₁-C₃ linéaire ou ramifié,
ou
R⁴ et R⁵ forment ensemble, avec l'atome d'azote qui les relie en tant que chaînon de cycle, un radical cycloaliphatique saturé ou insaturé, comportant éventuellement au moins un autre hétéroatome en tant que chaînon de cycle, qui peut être une ou plusieurs fois substitué, de façon identique ou différente, par un substituant choisi dans l'ensemble constitué par des groupes alkyle en C₁-C₅, -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N [alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué ; le substituant furannyle, thiophényle, pyridinyle, phényle ou benzyle respectif pouvant lui-même être non substitué ou une ou plusieurs fois substitué par des substituants choisis, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅ ;
les radicaux aliphatiques mentionnés précédemment pouvant être substitués par des substituants choisis, indépendamment les uns des autres, dans l'ensemble constitué par des atomes d'halogène, des groupes hydroxy, -CN, -CF₃, -CHF₂, -CH₂F, alcoxy en C₁-C₆, et phényle éventuellement au moins monosubstitué, dont les substituants peuvent être choisis chacun indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅ ;
les radicaux aryle mentionnés précédemment pouvant être substitués par des substituants choisis, indépendamment les uns des autres, dans l'ensemble constitué par des groupes alkyle en C₁-C₅, -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N [alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué, le substituant furannyle, thiophényle, pyridinyle, phényle ou benzyle respectif pouvant lui-même être non substitué ou une ou plusieurs fois substitué par des substituants choisis, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅ ;
les radicaux cycloaliphatiques mentionnés précédemment à la position des radicaux R⁴ et R⁵ pouvant être substitués par des substituants choisis, indépendamment les uns des autres, dans l'ensemble constitué par des groupes alkyle en C₁-C₅, -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N[alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué, le substituant furannyle, thiophényle, pyridinyle, phényle ou benzyle respectif pouvant lui-même être non substitué ou substitué par des substituants choisis, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅ ;
chaque fois éventuellement sous forme de leurs stéréoisomères purs, en particulier énantiomères ou diastéréoisomères, de leurs racémates ou sous forme de mélanges des stéréoisomères, en particulier des énantiomères et/ou diastéréoisomères, en un rapport quelconque de mélange, ou chaque fois sous forme de sels correspondants ou chaque fois sous forme de produits de solvatation correspondants,
étant entendu que sont exclus les composés de formule générale I dans lesquels
les radicaux R¹ et R² ainsi que les radicaux R⁴ et R⁵ représentent respectivement, ensemble avec l'atome d'azote qui les relie en tant que chaînon de cycle, le même radical choisi dans l'ensemble constitué par les groupes pyrrolidinyle, morpholinyle et pipéridinyle, et le radical R³ représente chaque fois le groupe méthyle, ainsi que
les radicaux R¹ et R² forment ensemble, avec l'atome d'azote qui les relie en tant que chaînon de cycle, un radical pipéridinyle et les radicaux R³ à R⁵ représentent chacun le groupe méthyle.

2. Composés selon la revendication 1, **caractérisés en ce que** les radicaux R¹ et R² forment ensemble, avec l'atome d'azote qui les relie en tant que chaînon de cycle, un radical cycloaliphatique saturé ou insaturé, à 4, 5, 6, 7, 8 ou 9 chaînons, comportant éventuellement au moins un autre hétéroatome en tant que chaînon de cycle, qui peut être une ou plusieurs fois substitué, de façon identique ou différente, par un substituant choisi dans l'ensemble constitué par des groupes alkyle en C₁-C₅, -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N[alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué,
de préférence forment ensemble, avec l'atome d'azote qui les relie en tant que chaînon de cycle, un radical cycloaliphatique saturé ou insaturé, à 4, 5, 6, 7, 8 ou 9 chaînons, comportant éventuellement en tant que chaînon de cycle au moins un autre hétéroatome choisi dans l'ensemble constitué par les atomes d'azote, d'oxygène, d'azote et de soufre, qui peut être une ou plusieurs fois substitué, de façon identique ou différente, par un substituant choisi dans l'ensemble constitué par des groupes alkyle en C₁-C₅, -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N[alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué,
de façon particulièrement préférée forment ensemble, avec l'atome d'azote qui les relie en tant que chaînon de cycle, un radical imidazolidinyle, aziridinyle, azétidinyle, pyrrolidinyle, pipéridinyle, azépanyle, azokanyle, pipérazinyle, morpholinyle ou thiomorpholinyle, qui peut être une ou plusieurs fois substitué, de façon identique ou différente, par un substituant choisi dans l'ensemble constitué par des groupes alkyle en C₁-C₅, -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N[alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué ;
le substituant furannyle, thiophényle, pyridinyle, phényle ou benzyle respectif pouvant lui-même être non substitué ou une ou plusieurs fois substitué par des substituants choisis, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅ ;

3. Composés selon la revendication 1 ou 2, **caractérisés en ce que** le radical R³ représente un radical aliphatique en C₁-C₁₀ linéaire ou ramifié, saturé ou insaturé, non substitué ou au moins monosubstitué, les substituants pouvant être choisis, indépendamment les uns des autres, dans l'ensemble constitué par des atomes d'halogène et des groupes hydroxy, -CN, -CF₃, -CHF₂, -CH₂F, alcoxy en C₁-C₆ et phényle éventuellement au moins monosubstitué,, dont les substituants peuvent être choisis chacun, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅,
de préférence un radical alkyle en C₁-C₅ linéaire ou ramifié, non substitué ou au moins monosubstitué, les substituants pouvant être choisis, indépendamment les uns des autres, dans l'ensemble constitué par des atomes d'halogène et des groupes hydroxy, -CN, -CF₃, -CHF₂, -CH₂F, alcoxy en C₁-C₆ et phényle éventuellement au moins monosubstitué, dont les substituants peuvent être choisis chacun, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅,
de façon particulièrement préférée un radical alkyle choisi dans l'ensemble constitué par les groupes méthyle, éthyle, n-propyle, isopropyle, n-butyle, isobutyle, sec-butyle et tert-butyle.

4. Composés selon une ou plusieurs des revendications 1 à 3, **caractérisés en ce que** le radical R⁴ représente un atome d'hydrogène, un radical aliphatique en C₁-C₁₀ linéaire ou ramifié, saturé ou insaturé, non substitué ou au moins monosubstitué, les substituants pouvant être choisis, indépendamment les uns des autres, dans l'ensemble constitué par des atomes d'halogène et des groupes hydroxy, -CN, -CF₃, -CHF₂, -CH₂F, alcoxy en C₁-C₆ et phényle éventuellement au moins monosubstitué, dont les substituants peuvent être choisis chacun, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅, un radical cycloaliphatique saturé ou insaturé à 3, 4, 5, 6, 7, 8 ou 9 chaînons, non substitué ou au moins monosubstitué, comportant éventuellement au moins un hétéroatome en tant que chaînon de cycle, qui peut être lié par un groupe alkylène en C₁-C₃ linéaire ou ramifié, ou un radical aryle à 5-14 chaînons, non substitué ou au moins monosubstitué, lié par un groupe alkylène en C₁-C₃ linéaire ou ramifié,
de préférence un atome d'hydrogène, un radical alkyle en C₁-C₅ linéaire ou ramifié, un radical cycloaliphatique saturé ou insaturé à 4, 5, 6, 7, 8 ou 9 chaînons, qui peut être lié par un groupe alkylène en C₁-C₃ linéaire ou ramifié, ou un radical phényle non substitué ou au moins monosubstitué, lié par un groupe alkylène en C₁-C₃ linéaire ou ramifié,
de façon particulièrement préférée un atome d'hydrogène, un radical alkyle choisi dans l'ensemble constitué par les groupes méthyle, éthyle, propyle, isopropyle, n-butyle, isobutyle, sec-butyle et tert-butyle ou un radical benzyle ;
les radicaux aryle mentionnés précédemment pouvant être substitués par des substituants choisis, indépendamment les uns des autres, dans l'ensemble constitué par des groupes alkyle en C₁-C₅, -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N[alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué, le substituant furannyle, thiophényle, pyridinyle, phényle ou benzyle respectif pouvant lui-même être non substitué ou une ou plusieurs fois substitué par des substituants choisis, indépendamment les uns des autres, dans l'ensemble constitué par hydroxy, F, Cl, Br, I, -NH₂, -NH-alkyle(C₁-C₅), -N [alkyle(C₁-C₅)]₂, -NO₂, -CN, -CF₃, -CHF₂, -CH₂F, alkyle en C₁-C₅ et alcoxy en C₁-C₅ ;
les radicaux cycloaliphatiques mentionnés précédemment pouvant être substitués par des substituants choisis, indépendamment les uns des autres, dans l'ensemble constitué par des groupes alkyle en C₁-C₅, -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N[alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué, le substituant furannyle, thiophényle, pyridinyle, phényle ou benzyle respectif pouvant lui-même être non substitué ou substitué par des substituants choisis, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅.

5. Composés selon une ou plusieurs des revendications 1 à 4, **caractérisés en ce que** le radical R⁵ représente un radical aliphatique en C₁-C₁₀ linéaire ou ramifié, saturé ou insaturé, non substitué ou au moins monosubstitué, les substituants pouvant être choisis, indépendamment les uns des autres, dans l'ensemble constitué par des atomes d'halogène et des groupes hydroxy, -CN, -CF₃, -CHF₂, -CH₂F, alcoxy en C₁-C₆ et phényle éventuellement au moins monosubstitué, dont les substituants peuvent être choisis chacun, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅, un radical cycloaliphatique saturé ou insaturé à 3, 4, 5, 6, 7, 8 ou 9 chaînons, non substitué ou au moins monosubstitué, comportant éventuellement au moins un hétéroatome en tant que chaînon de cycle, qui peut être lié par un groupe alkylène en C₁-C₃ linéaire ou ramifié, ou un radical aryle à 5-14 chaînons, non substitué ou au moins monosubstitué, lié par un groupe alkylène en C₁-C₃ linéaire ou ramifié,
de préférence un radical alkyle en C₁-C₅ linéaire ou ramifié, un radical cycloaliphatique saturé ou insaturé à 4, 5, 6, 7, 8 ou 9 chaînons, qui peut être lié par un groupe alkylène en C₁-C₃ linéaire ou ramifié, ou un radical phényle non substitué ou au moins monosubstitué, lié par un groupe alkylène en C₁-C₃ linéaire ou ramifié,
de façon particulièrement préférée un radical alkyle choisi dans l'ensemble constitué par les groupes méthyle, éthyle, propyle, isopropyle, n-butyle, isobutyle, sec-butyle et tert-butyle ou un radical benzyle ;
les radicaux aryle mentionnés précédemment pouvant être substitués par des substituants choisis, indépendamment les uns des autres, dans l'ensemble constitué par des groupes alkyle en C₁-C₅, -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅) -N[alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué, le substituant furannyle, thiophényle, pyridinyle, phényle ou benzyle respectif pouvant lui-même être non substitué ou une ou plusieurs fois substitué par des substituants choisis, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅ ;
les radicaux cycloaliphatiques mentionnés précédemment pouvant être substitués par des substituants choisis, indépendamment les uns des autres, dans l'ensemble constitué par des groupes alkyle en C₁-C₅, -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N[alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué, le substituant furannyle, thiophényle, pyridinyle, phényle ou benzyle respectif pouvant lui-même être non substitué ou substitué par des substituants choisis, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅.

6. Composés selon une ou plusieurs des revendications 1 à 5, **caractérisés en ce que** les radicaux R⁴ et R⁵ forment ensemble, avec l'atome d'azote qui les relie en tant que chaînon de cycle, un radical cycloaliphatique saturé ou insaturé, à 4, 5, 6, 7, 8 ou 9 chaînons, comportant éventuellement au moins un autre hétéroatome en tant que chaînon de cycle, qui peut être une ou plusieurs fois substitué, de façon identique ou différente, par un substituant choisi dans l'ensemble constitué par des groupes alkyle en C₁-C₅, -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N[alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué,
de préférence forment ensemble, avec l'atome d'azote qui les relie en tant que chaînon de cycle, un radical cycloaliphatique saturé ou insaturé, à 4, 5, 6, 7, 8 ou 9 chaînons, comportant éventuellement en tant que chaînon de cycle au moins un autre hétéroatome choisi dans l'ensemble constitué par les atomes d'azote, d'oxygène et de soufre, qui peut être une ou plusieurs fois substitué, de façon identique ou différente, par un substituant choisi dans l'ensemble constitué par des groupes alkyle en C₁-C₅, -C(=O)-O-alkyle(C₁-C₅), -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N[alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué,
de façon particulièrement préférée forment ensemble, avec l'atome d'azote qui les relie en tant que chaînon de cycle, un radical imidazolidinyle, aziridinyle, azétidinyle, pyrrolidinyle, pipéridinyle, azépanyle, azokanyle, pipérazinyle, morpholinyle ou thiomorpholinyle, qui peut être une ou plusieurs fois substitué, de façon identique ou différente, par un substituant choisi dans l'ensemble constitué par des groupes alkyle en C₁-C₅, -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N[alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué ;
le substituant furannyle, thiophényle, pyridinyle, phényle ou benzyle respectif pouvant lui-même être non substitué ou une ou plusieurs fois substitué par des substituants choisis, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅ ;

7. Composés selon une ou plusieurs des revendications 1 à 6, **caractérisés en ce que**
les radicaux R¹ et R² forment ensemble, avec l'atome d'azote qui les relie en tant que chaînon de cycle, un radical cycloaliphatique saturé ou insaturé, à 4, 5, 6, 7, 8 ou 9 chaînons, comportant éventuellement en tant que chaînon de cycle au moins un autre hétéroatome choisi dans l'ensemble constitué par les atomes d'azote, d'oxygène et de soufre, qui peut être une ou plusieurs fois substitué, de façon identique ou différente, par un substituant choisi dans l'ensemble constitué par des groupes alkyle en C₁-C₅, -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N[alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué ; le substituant furannyle, thiophényle, pyridinyle, phényle ou benzyle respectif pouvant lui-même être non substitué ou une ou plusieurs fois substitué par des substituants choisis, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅.
le radical R³ représente un radical alkyle en C₁-C₅ linéaire ou ramifié, non substitué ou au moins monosubstitué, les substituants étant choisis, indépendamment les uns des autres, dans l'ensemble constitué par des atomes d'halogène et des groupes hydroxy, -CN, -CF₃, -CHF₂, -CH₂F, alcoxy en C₁-C₆ et phényle éventuellement au moins monosubstitué, dont les substituants peuvent être choisis chacun, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅,
le radical R⁴ représente un atome d'hydrogène, un radical alkyle en C₁-C₅ linéaire ou ramifié, un radical cycloaliphatique saturé ou insaturé à 4, 5, 6, 7, 8 ou 9 chaînons, qui peut être lié par un groupe alkylène en C₁-C₃ linéaire ou ramifié, ou un radical phényle non substitué ou au moins monosubstitué, lié par un groupe alkylène en C₁-C₃ linéaire ou ramifié,
le radical R⁵ représente un radical alkyle en C₁-C₅ linéaire ou ramifié, un radical cycloaliphatique saturé ou insaturé à 4, 5, 6, 7, 8 ou 9 chaînons, qui peut être lié par un groupe alkylène en C₁-C₃ linéaire ou ramifié, ou un radical phényle non substitué ou au moins monosubstitué, lié par un groupe alkylène en C₁-C₃ linéaire ou ramifié, ou
les radicaux R⁴ et R⁵ forment ensemble, avec l'atome d'azote qui les relie en tant que chaînon de cycle, un radical cycloaliphatique saturé ou insaturé à 4, 5, 6, 7, 8 ou 9 chaînons, comportant éventuellement en tant que chaînon de cycle au moins un autre hétéroatome choisi dans l'ensemble constitué par les atomes d'azote, d'oxygène et de soufre, qui peut être une ou plusieurs fois substitué, de façon identique ou différente, par un substituant choisi dans l'ensemble constitué par des groupes alkyle en C₁-C₅, -C(=O)-O-alkyle(C₁-C₅), -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N[alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué ; le substituant furannyle, thiophényle, pyridinyle, phényle ou benzyle respectif pouvant lui-même être non substitué ou une ou plusieurs fois substitué par des substituants choisis, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅ ;
les radicaux aryle mentionnés précédemment pouvant être substitués par des substituants choisis, indépendamment les uns des autres, dans l'ensemble constitué par des groupes alkyle en C₁-C₅, -C(=O)-O-alkyle(C₁-C₅), -O-alkyle(C₁-C₅), Cl, F, Br, I, -C(=O)-alkyle(C₁-C₅), -NH₂, -NH-alkyle(C₁-C₅), -N[alkyle(C₁-C₅)]₂, furannyle éventuellement au moins monosubstitué, thiophényle éventuellement au moins monosubstitué, pyridinyle éventuellement au moins monosubstitué, phényle éventuellement au moins monosubstitué et benzyle éventuellement au moins monosubstitué, le substituant furannyle, thiophényle, pyridinyle, phényle ou benzyle respectif pouvant lui-même être non substitué ou une ou plusieurs fois substitué par des substituants choisis, indépendamment les uns des autres, dans l'ensemble constitué par hydroxy, F, Cl, Br, I, -NH₂, -NH-alkyle(C₁-C₅), -N[alkyle(C₁-C₅)]₂, -NO₂, -CN, -CF₃, -CHF₂, -CH₂F, alkyle en C₁-C₅ et alcoxy en C₁-C₅.

8. Composés selon une ou plusieurs des revendications 1 à 7, **caractérisés en ce que**
R¹ et R² forment ensemble, avec l'atome d'azote qui les relie en tant que chaînon de cycle, un radical imidazolidinyle, aziridinyle, azétidinyle, pyrrolidinyle, pipéridinyle, azépanyle, azokanyle, pipérazinyle, morpholinyle ou thiomorpholinyle, qui peut être substitué par un substituant choisi dans l'ensemble constitué par les groupes méthyle, éthyle, n-propyle, isopropyle, n-butyle, isobutyle, sec-butyle, tert-butyle, phényle et benzyle,
R³ représente un radical alkyle choisis dans l'ensemble constitué par les groupes méthyle, éthyle, n-propyle, isopropyle, n-butyle, isobutyle, sec-butyle et tert-butyle,
R⁴ représente un atome d'hydrogène, un radical alkyle choisi dans l'ensemble constitué par les groupes méthyle, éthyle, n-propyle, isopropyle, n-butyle, isobutyle, sec-butyle, tert-butyle, n-pentyle, isopentyle et néopentyle, ou représente un groupe benzyle,
R⁵ représente un radical alkyle choisi dans l'ensemble constitué par les groupes méthyle, éthyle, n-propyle, isopropyle, n-butyle, isobutyle, sec-butyle, tert-butyle, n-pentyle, isopentyle et néopentyle, ou représente un groupe benzyle,
ou
R⁴ et R⁵ forment ensemble, avec l'atome d'azote qui les relie en tant que chaînon de cycle, un radical imidazolidinyle, aziridinyle, azétidinyle, pyrrolidinyle, pipéridinyle, azépanyle, azokanyle, pipérazinyle, morpholinyle ou thiomorpholinyle, qui peut être substitué par un substituant choisi dans l'ensemble constitué par les groupes méthyle, éthyle, n-propyle, isopropyle, n-butyle, isobutyle, sec-butyle, tert-butyle, phényle et benzyle.

9. Composés selon une ou plusieurs des revendications 1 à 8, choisis parmi
la diéthyl-(1-méthyl-5-pipéridin-1-ylméthyl-1H-pyrrol-2-ylméthyl)-amine,
la 1-(1-méthyl-5-pipéridin-1-ylméthyl-1H-pyrrol-2-yl-méthyl)-4-méthylpipéridine,
la 4-[1-méthyl-5-(4-phényl-pipéridin-1-ylméthyl)-1H-pyrrol-2-ylméthyl]-morpholine,
la 1-(1-méthyl-5-pipéridin-1-ylméthyl-1H-pyrrol-2-yl-méthyl)-4-phényl-pipérazine,
le 1-[5-(4-benzyl-pipéridin-1-ylméthyl)-1-méthyl-1H-pyrrol-2-ylméthyl]-azépane,
la benzyl-[5-(4-benzyl-pipéridin-1-ylméthyl)-1-méthyl-1H-pyrrol-2-ylméthyl]-méthylamine,
la 4-[5-(4-benzyl-pipéridin-1-ylméthyl)-1-méthyl-1H-pyrrol-2-ylméthyl]-morpholine,
la (5-azépan-1-ylméthyl-1-méthyl-1H-pyrrol-2-ylméthyl)-benzyl-méthylamine,
la benzyl-méthyl-[1-méthyl-5-(4-phényl-pipéridin-1-yl-méthyl)-1H-pyrrol-2-ylméthyl]-amine et
la benzyl-méthyl-[1-méthyl-5-(4-phényl-pipéridin-1-yl-méthyl)-1H-pyrrol-2-ylméthyl]-amine,
chaque fois éventuellement sous forme de leurs stéréoisomères purs, en particulier énantiomères ou diastéréoisomères, de leurs racémates ou sous forme de mélanges des stéréoisomères, en particulier des énantiomères et/ou diastéréoisomères, en un rapport quelconque de mélange, ou chaque fois sous forme de sels correspondants ou chaque fois sous forme de produits de solvatation correspondants.

10. Procédé pour la préparation de composés 2,5-diaminométhyl-1H-pyrrole de formule générale I selon une ou plusieurs des revendications 1 à 9, **caractérisé en ce qu'**on fait réagir un composé de formule générale II, 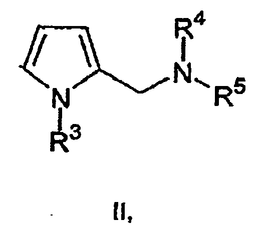 dans laquelle les radicaux R³, R⁴ et R⁵ ont les significations selon une ou plusieurs des revendications 1 à 9, avec un sel d'iminium de formule générale III, 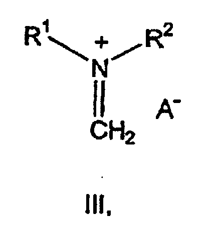 dans laquelle les radicaux R¹ et R² ont les significations selon une ou plusieurs des revendications 1 à 9, et A⁻ représente un anion convenable, de préférence Cl⁻, AlCl₄⁻, Br⁻, I⁻ ou CF₃SO₃⁻ (anion) triflate) et éventuellement on purifie le composé ainsi obtenu et éventuellement on l'isole.

11. Médicament contenant au moins un composé selon une ou plusieurs des revendications 1 à 9, y compris les composés exclus, ainsi qu'éventuellement un ou plusieurs adjuvants physiologiquement acceptables.

12. Médicament selon la revendication 11, pour la régulation, de préférence l'inhibition, de la capture de la 5-hydroxytryptamine (5-HT).

13. Médicament selon la revendication 11, pour la régulation, de préférence l'inhibition, de la capture de la noradrénaline (NA).

14. Médicament selon la revendication 11, pour la régulation des récepteurs d'opiacés, de préférence pour la régulation des récepteurs d'opiacés p.

15. Médicament selon une ou plusieurs des revendications 11 à 14, destiné à la prophylaxie et/ou au traitement de troubles ou de maladies qui sont causés au moins en partie par les récepteurs de 5-hydroxytryptamine (5-HT) et/ou les récepteurs de noradrénaline (NA) et/ou les récepteurs d'opiacés, en particulier les récepteurs d'opiacés p.

16. Médicament selon une ou plusieurs des revendications 11 à 15, destiné au traitement de douleurs.

17. Médicament selon la revendications 16, destiné au traitement de douleurs choisies parmi les douleurs aiguës, les douleurs chroniques et les douleurs névropathiques.

18. Médicament selon une ou plusieurs des revendications 11 à 15, destiné à la prophylaxie et/ou au traitement de manifestations inflammatoires, de troubles de la mémoire, de maladies neurodégénératives, choisies de préférence dans l'ensemble constitué par la maladie de Parkinson, la chorée de Huntington, la maladie d'Alzheimer et la sclérose en plaques, l'épilepsie, de troubles cardiovasculaires, d'affections relatives à la rétention d'eau, de la motilité intestinale (diarrhée), de l'incontinence urinaire, de l'anorexie, de l'acouphène, du prurit, des dépressions, des dysfonctionnements sexuels, de préférence du dysfonctionnement érectile ou d'affections des voies respiratoires, de troubles de la prise d'aliments, choisis de préférence dans le groupe constitué par l'obésité, la boulimie, l'anorexie, la cachexie et le diabète de type II (diabète non insulinodépendant), ou destiné à l'anxiolyse, à la diurèse, à la répression du réflexe de miction, à la réduction du potentiel de dépendance aux opiacés, à la morphine de préférence, destiné à la modulation de l'activité motrice, à influer sur le système cardiovasculaire, de préférence à la vasodilatation des artères, ou à la régulation du bilan électrolytique.

19. Utilisation d'au moins un composé selon une ou plusieurs des revendications 1 à 9, à l'inclusion des composés exclus, pour la fabrication d'un médicament destiné à la régulation de la capture de la 5-hydroxytryptamine (5-HT), de préférence à l'inhibition de la capture de la 5-hydroxytryptamine (5-HT).

20. Utilisation d'au moins un composé selon une ou plusieurs des revendications 1 à 9, à l'inclusion des composés exclus, pour la fabrication d'un médicament destiné à la régulation de la capture de la noradrénaline (NA), de préférence à l'inhibition de la capture de la noradrénaline (NA).

21. Utilisation d'au moins un composé selon une ou plusieurs des revendications 1 à 9, à l'inclusion des composés exclus, pour la fabrication d'un médicament destiné à la régulation des récepteurs d'opiacés, de préférence à la régulation des récepteurs d'opiacés p.

22. Utilisation d'au moins un composé selon une ou plusieurs des revendications 1 à 9, à l'inclusion des composés exclus, pour la fabrication d'un médicament destiné à la prophylaxie et/ou au traitement de troubles ou de maladies qui sont causés au moins en partie par les récepteurs de 5-hydroxytryptamine (5-HT) et/ou les récepteurs de noradrénaline (NA) et/ou les récepteurs d'opiacés, en particulier les récepteurs d'opiacés µ.

23. Utilisation d'au moins un composé selon une ou plusieurs des revendications 1 à 9, à l'inclusion des composés exclus, pour la fabrication d'un médicament destiné au traitement de douleurs, de préférence de douleurs choisies dans le groupe constitué par les douleurs aiguës, les douleurs chroniques et les douleurs névropathiques.

24. Utilisation d'au moins un composé selon une ou plusieurs des revendications 1 à 9, à l'inclusion des composés exclus, pour la fabrication d'un médicament destiné à la prophylaxie et/ou au traitement de manifestations inflammatoires, de troubles de la mémoire, de maladies neurodégénératives, choisies de préférence dans l'ensemble constitué par la maladie de Parkinson, la chorée de Huntington, la maladie d'Alzheimer et la sclérose en plaques, l'épilepsie, de troubles cardiovasculaires, d'affections relatives à la rétention d'eau, de la motilité intestinale (diarrhée), de l'incontinence urinaire, de l'anorexie, de l'acouphène, du prurit, des dépressions, des dysfonctionnements sexuels, de préférence du dysfonctionnement érectile ou d'affections des voies respiratoires, de troubles de la prise d'aliments, choisis de préférence dans le groupe constitué par l'obésité, la boulimie, l'anorexie, la cachexie et le diabète de type II (diabète non insulinodépendant), ou destiné à l'anxiolyse, à la diurèse, à la répression du réflexe de miction, à la réduction du potentiel de dépendance aux opiacés, à la morphine de préférence, destiné à la modulation de l'activité motrice, à influer sur le système cardiovasculaire, de préférence à la vasodilatation des artères, ou à la régulation du bilan électrolytique.
